# FASCICULE DE BREVET EUROPEEN

(11) **EP 2 181 113 B1**
(45) Date de publication et mention de la délivrance du brevet: **18.11.2015**
(21) Numéro de dépôt: 08828311.4
(22) Date de dépôt: 15.07.2008
(51) Int. Cl.: C07D 493/10

(54) **PROCEDE DE PREPARATION de PHTALEINES POLYCARBOXYLEES UTILISEES COMME COLORANTS FLUORESCENTS**
HERSTELLUNGSVERFAHREN von POLYCARBOXYLAT-PHTHALEINE ALS FLUORESZIERENDE FARBSTOFFE
METHOD FOR THE PREPARATION of POLYCARBOXYLATE PHTALEINS USED AS FLUORESCENT DYES

(30) Priorité: 13.07.2007 FR 0756497
(43) Date de publication de la demande: 05.05.2010
(73) Titulaire: Laboratoires Synth-Innove, F-75020 Paris (FR)
(72) Inventeur: TRAN-GUYON, Joanne, F-78180 Montigny le Bretonneux (FR); SCHERNINSKI, François, F-75011 Paris (FR); GUYON, Vincent, F-78180 Montigny le Bretonneux (FR)
(74) Mandataire: Regimbeau
(86) Numéro de dépôt international: PCT/FR2008/001033
(87) Numéro de publication internationale: WO 2009/027604

(56) Documents cités:
- WO-A-2007/017602
- WO-A2-03/025192
- FR-A- 2 846 331
- US-A- 4 351 760
- US-A1- 2006 094 053
- LIU, JIXIANG ET AL: "Fluorescent molecular probes. III. 2',7'-Bis-(3-carboxypropyl)-5-(and-6)- carboxyfluorescein (BCPCF): a new polar dual-excitation and dual-emission pH indicator with a pKa of 7.0" BIOORGANIC & MEDICINAL CHEMISTRY LETTERS , 7(23), 3069-3072 CODEN: BMCLE8; ISSN: 0960-894X, 1997, XP004136586

## Description

La présente invention concerne un procédé de préparation de composés polycarboxyliques utilisables comme colorants fluorescents.

Les colorants fluorescents sont utilisés dans nombreux domaines en tant qu'indicateurs biologiques. Une application particulièrement intéressante des colorants fluorescents est leur utilisation en tant que sondes de pH. Par le terme «sonde de pH» on entend un colorant fluorescent permettant la mesure du pH dans un milieu biologique *in vitro* et *in vivo.*

La détermination précise du pH tissulaire en médecine humaine et en biotechnologie nécessite une sonde de pH dont les caractéristiques spectrales d'absorption et de fluorescence soient constantes, parfaitement maîtrisées et reproductibles car l'étalonnage de la mesure de pH est principalement basé sur l'expérience clinique. Ceci suppose une détermination du pH à partir d'une seule structure moléculaire et non pas d'un mélange d'isomères. De plus, les exigences de qualité et de sécurité des nouvelles normes pharmaceutiques internationales (ICH : International Commission of Harmonisation, ICH Q Topic Q3A 1999) se sont considérablement accrues et la fabrication de médicaments de diagnostic à partir de colorants polycarboxyliques fluorescents requiert maintenant un niveau de pureté élevé surtout lorsqu'ils sont administrés par voie intraveineuse chez l'homme. Par ailleurs, l'évaluation de l'innocuité d'un agent de diagnostic et le suivi de sa pharmacovigilance ne sont concrètement envisageables que s'il est administré sous forme d'un isomère pur ; ces deux évaluations étant nécessaires pour la délivrance de son autorisation de mise sur le marché.

Du fait de la multiplicité de leurs fonctions acides carboxyliques les colorants fluorescents polycarboxyliques n'étaient pas accessibles sous forme d'un seul isomère par les méthodes de synthèse de l'art antérieur et ne pouvaient être obtenus que sous forme d'un mélange en proportions variables de plusieurs isomères de position dont les caractéristiques spectrales étaient différentes. Cette inhomogénéité spectrale empêchait l'utilisation diagnostique des colorants polycarboxyliques pour la détermination du pH tissulaire chez l'homme. L'apparition d'isomères de position est notamment due au fait que les colorants polycarboxyliques fluorescents fabriqués selon les techniques de l'art antérieur, sont obtenus par une réaction unique de condensation qui doit mettre en jeu certaines fonctions acides carboxyliques des réactifs. Néanmoins, les autres fonctions acides carboxyliques des réactifs, pouvant être également activées durant la réaction, réagissent de façon concurrente et génèrent la formation des isomères de position. Ainsi, la préparation des colorants polycarboxyliques fluorescents selon les procédés de synthèse de l'art antérieur qui ne comportent qu'une seule étape de condensation, favorise l'apparition de nombreux sous-produits, diminue le rendement de la réaction et génère de nombreuses impuretés.

Ainsi il est connu de préparer certains des colorants fluorescents polycarboxyliques comme la Bis-carboxyéthyl-carboxyfluorescéine en une seule étape de condensation par chauffage à la température de fusion d'un mélange d'un dérivé de l'acide trimellitique et d'un dérivé phénolique, dans les proportions souhaitées éventuellement en présence d'un catalyseur de Lewis en particulier le chlorure de zinc. Toutefois, cette réaction de condensation n'est pas spécifique et génère de nombreux produits secondaires et notamment un mélange en proportions variables des isomères de positions. De plus, le rendement de cette réaction est généralement faible.

Les isomères de position des colorants polycarboxyliques fluorescents ne possèdent pas les mêmes propriétés spectrales mais leur solubilité et leur polarité sont très voisines. Cette particularité empêche leur séparation physique par recristallisation ou même par chromatographie. Il s'en suit que les procédés de séparation et de purification connus de l'art antérieur ne permettent pas davantage l'obtention d'un composé sous forme d'un seul de ses isomères de position, c'est-à-dire avec une pureté isomérique élevée.

Par exemple, la Bis-carboxyéthyl-carboxyfluorescéine ou BCECF est connue pour la mesure *in vitro* du pH par fluorimétrie en utilisant deux longueurs d'ondes d'excitation et une longueur d'onde d'émission. La BCECF produite selon les techniques de l'art antérieur est un mélange de plusieurs isomères de position dont les principaux composants sont la 2',7'-Bis(2-carboxyéthyl)-5-carboxyfluorescéine et la 2',7'-Bis(2-carboxyéthyl)-6-carboxyfluorescéine, ce qui ne permet pas son utilisation *in vivo* en imagerie médicale, notamment chez l'homme.

Or il existe actuellement un besoin important sur le plan médical pour des colorants fluorescents polycarboxyliques de pureté isomérique élevée.

Ainsi la forme mono-isomérique (mono-isomère en position 5 ou mono-isomère en position 6) de la BCECF présenterait un grand intérêt en réanimation et en ophtalmologie pour la réalisation d'imagerie médicale en fluorescence, notamment au cours d'endoscopies digestives et d'angiographies de la rétine. Ce nouveau type d'imagerie médicale permettrait d'obtenir une cartographie de pH tissulaire de grande valeur diagnostique et pronostique par exemple pour la prise en charge thérapeutique du sepsis et pour le traitement des maladies micro-vasculaires de l'oeil (par exemple la dégénérescence maculaire liée à l'âge).

Par ailleurs, l'intérêt diagnostique des colorants fluorescents polycarboxyliques de pureté isomérique élevée est actuellement accru par l'apparition de nouveaux médicaments efficaces permettant le traitement des pathologies micro-vasculaires de l'oeil et de produits issus des biotechnologies permettant le traitement du sepsis. Ces colorants polycarboxyliques peuvent être utilisés pour l'obtention d'une imagerie médicale fonctionnelle permettant de visualiser l'anoxie et/ou la souffrance tissulaire par la réalisation d'une cartographie du pH des tissus.

Ainsi, le brevet US 4,351,760 décrit des composés conjugués antigéniques fluorescents, et cite des composés de formule (I) selon la présente invention, mais ne donne aucune indication quant à la pureté ou à la préparation de tels composés.

Par ailleurs, l'article de Liu et al (Bioorganic & Medicinal Chemistry Letters, 1997, vol 7, 23, pp 3069-3072) se rapporte à la BCPCF, et décrit une voie de synthèse de ce composé.

Enfin, la demande FR 2 846 331 porte sur des phtaléines de pureté élevée. Ce document décrit un procédé de préparation de ces phtaléines consistant en une condensation d'un anhydride phtalique avec un dérivé naphthol ou phénol suivis d'une conversion par action d'un acide fort en milieu anhydre.

Les inventeurs, à l'issue de recherches approfondies, ont mis au point un procédé de préparation de colorants fluorescents polycarboxyliques permettant une mesure du pH et présentant une pureté isomérique de position minimum de 95 %, exprimée en poids d'un seul isomère de position du colorant fluorescent polycarboxylique.

Au sens de la présente invention, une pureté isomérique (de position) égale à au moins 95% signifie que l'isomère majoritaire représente 95% en poids relativement à l'ensemble des isomères de position ; une pureté isomérique égale à au moins 98% signifie que l'isomère majoritaire représente 98% en poids relativement à l'ensemble des isomères de position et une pureté isomérique égale à au moins 99% signifie que l'isomère majoritaire représente 99% en poids relativement à l'ensemble des isomères de position.

Aussi la présente invention décrit-elle des composés de pureté isomérique égale à au moins 95%, avantageusement égale à au moins 98%, encore plus avantageusement égale à au moins 99% répondant à la formule (I) 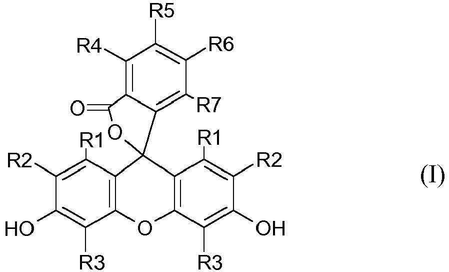 dans laquelle :
- deux au moins des R1, R2, R3, R4, R5, R6 et R7 représentent indépendamment l'un de l'autre un groupement -A-COOH identique ou différent ou un de ses sels alcalins ;
- deux au moins des groupements A parmi tous les groupements A du composé (I) n'étant pas une simple liaison, où A représente:
   ∘ soit un groupe -(CH₂)ₙ-, n étant un nombre entier de 0 à 30 et n étant différent de 0 pour au moins un des groupements A,
   ∘ soit un groupe alkylène en C₁ à C₃₀ éventuellement ramifiés et/ou pouvant comprendre une ou plusieurs double(s) ou triple(s) liaison(s), éventuellement substitué par un ou plusieurs groupe(s) choisi(s) parmi halogéno, hydroxy, -CONHR,-CONR10R11 où R, R10 et R11 représentent chacun indépendamment les uns des autres un groupe alkyle en C₁ à C₃₀,
   ∘ soit un groupe cycloalkylène en C₃ à C₁₂,
   ∘ soit un groupe alkyle en (C₁ à C₃₀)cycloalkylène en (C₃ à C₁₂),
   ∘ soit un groupe arylène éventuellement substitué par un ou plusieurs groupes choisis parmi les halogènes et les hydroxyles,
   ∘ soit un groupe alkyle en (C₁ à C₃₀)arylène, éventuellement substitué par un ou plusieurs groupes choisis parmi les halogènes et les hydroxyles,
   ∘ soit un groupe aryl(alkyle en C₁ à C₃₀) éventuellement substitué par un ou plusieurs groupes choisis parmi les halogènes et les hydroxyles, et
- tous les autres R1, R2, R3, R4, R5, R6 et R7, identiques ou différents les uns des autres, sont choisis indépendamment les uns des autres, dans le groupe comprenant:
   ∘ l'hydrogène,
   ∘ les atomes d'halogène,
   ∘ le groupe hydroxy,
   ∘ le groupe amino,
   ∘ le groupe phosphate,
   ∘ le groupe nitro,
   ∘ le groupe sulfonate,
   ∘ les alkyles en C₁ à C₃₀, éventuellement ramifiés et/ou pouvant comprendre une ou plusieurs double(s) ou triple(s) liaison(s), éventuellement substitués par un ou plusieurs substituants choisis parmi halogéno, hydroxy, nitro, amino, sulfo, -CONHR et - CONR11R12 avec R, R11 et R12 représentant chacun indépendamment un groupe alkyle en C₁ à C₃₀,
   ∘ les cycloalkyle en C₃ à C₁₂,
   ∘ les alkyloxy en C₁ à C₃₀,
   ∘ les aryles éventuellement substitués par un ou plusieurs groupes choisis parmi les groupements halogèno, hydroxyles,
   ∘ les aryloxy,
   ∘ les aryl(alkyle en C₁ à C₃₀),
   ∘ les bases puriques ou pyrimidiques,
   ∘ les oses,
   ∘ les oligosides,
   ∘ les polyosides,
   ∘ les polypeptides,
   ∘ les protéines,
   ∘ les phospholipides,
et les esters correspondants, notamment comme pro-médicaments.

Au sens de la présente invention, on entend par sels alcalins du groupe -A-COOH, notamment les sels de sodium et de potassium.

Au sens de la présente invention, on entend par «simple liaison» une simple liaison chimique covalente.

Au sens de la présente invention, on entend par alkyle en C₁ à C₃₀ tout groupe alkyle de 1 à 30 atomes de carbones, linéaire ou ramifié, en particulier les groupes méthyle, éthyle, n-propyle, iso-propyle, n-butyle, iso-butyle, t-butyle, *n*-pentyle, isopentyle, hexyle, heptyle, octyle nonyle, dodecyle, et les groupes alkyles en C₁₁ à C₂₀.

Au sens de la présente invention, on entend par «aryle», un ou plusieurs cycles aromatiques ayant 5 à 8 atomes de carbones, pouvant être accolés ou fusionnés. En particulier, les groupes aryles peuvent être des groupes monocycliques ou bicycliques, comme par exemple phényle, naphtyle, tétrahydronaphtyle ou indanyle et par aryl(alkyle en C₁ à C₃₀) ces mêmes groupes liés à un groupe alkyle comprenant de 1 à 30 atomes de carbone. On peut citer à titre d'exemple le groupe benzyle.

Au sens de la présente invention, on entend par «ester», un groupe - COOR20 où R20 représente un groupe alkyle en C₁ à C₃₀ ou aryle tels qu'ils ont été définis précédemment.

Au sens de la présente invention, on entend par «base purique», notamment la purine, l'adénine et la guanine.

Au sens de la présente invention, on entend par «base pyrimidique», notamment la pyrimidine, la cytosine, l'uracile et la thymine.

Au sens de la présente invention, on entend par «ose», un monomère de glucides, appelé également monosaccharide. On peut citer à titre d'exemple, les trioses, les tétroses, les pentoses comme le ribose, l'arabinose et le xylose, et les hexoses comme le glucose, le mannose et le galactose.

Au sens de la présente invention, on entend par «polyoside», un polymère composé de plusieurs monosaccharides définis ci-dessus liés entre eux par une liaison osidique. On peut citer à titre d'exemple, le dextran.

Au sens de la présente invention, on entend par «oligoside» un polymère composé de 3 à 6 monosaccharides définis ci-dessus liés entre eux par une liaison osidique.

Au sens de la présente invention, on entend par «polypeptide» une chaîne comprenant moins de 100 acides aminés reliés par des liaisons peptidiques, de préférence les chaînes peptidiques contenant moins de 10 acides aminés, ainsi on peut citer de manière avantageuse les pentapeptides.

Au sens de la présente invention, on entend par «protéine» une chaîne comprenant plus de 100 acides aminés reliés par des liaisons peptidiques. On peut citer de manière avantageuse l'albumine et les immunoglobulines.

Au sens de la présente invention les phospholipides sont choisis dans le groupe comprenant notamment la phosphatidyl sérine, le phosphatidyl inositol, la phosphatidyl éthanolamine, la phosphatidyl choline et la sphyngomyéline.

L'invention décrit les composés répondant à la formule (Ia) : 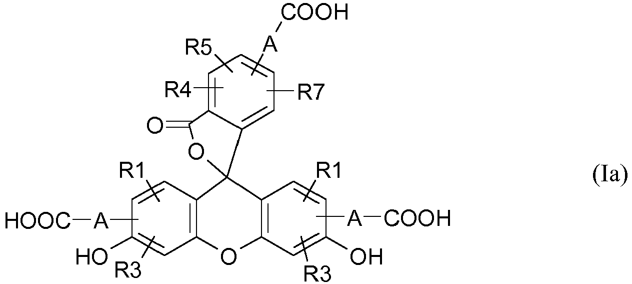 dans lesquelles A et R1, R3, R4, R5, R7 différents de -A-COOH sont tels que définis pour le composé de formule(I).

L'invention décrit également les composés choisis parmi les isomères de position répondant à l'une des formules (Ia1) à (Ia4) qui suivent : 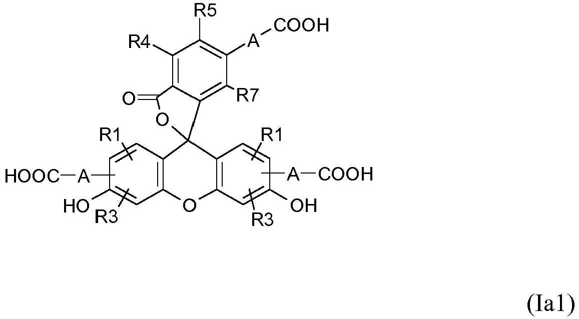 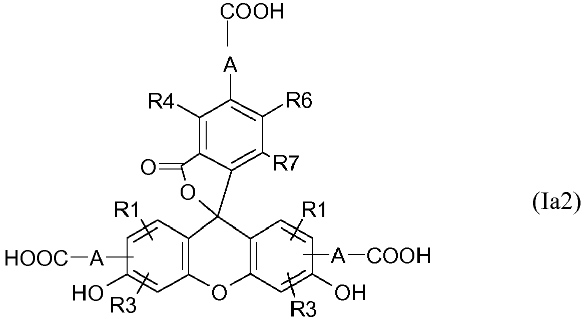 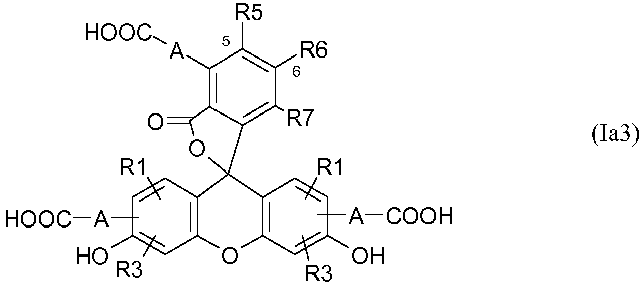 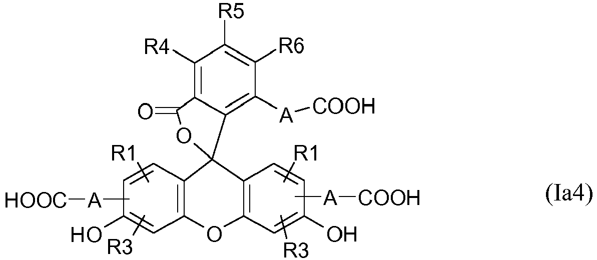 dans lesquelles A et R1, R3, R4, R5, R6 différents de -A-COOH sont tels que définis pour le composé de formule(Ia).

L'invention décrit également les composés répondant à la formule (Ib) 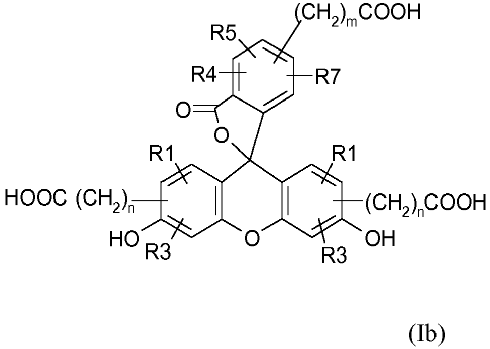 dans laquelle n et m sont des nombres entiers de 0 à 30, avantageusement de 0 à 10, encore plus avantageusement de 0 à 3 identiques ou différents, l'un au moins de n et m étant différent de 0 et R1, R3, R4, R5, R7 différents de -A-COOH sont tels que définis précédemment pour le composé de formule(I).

Avantageusement les composés décrits sont choisis parmi les isomères de position répondant à l'une des formules (Ib1) à (Ib4) qui suivent: 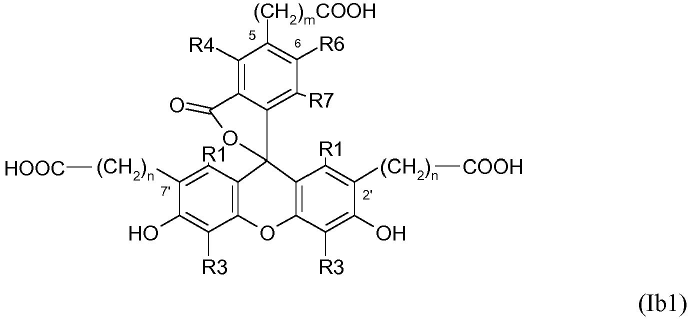 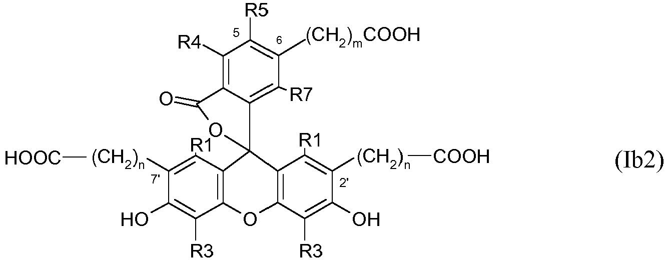 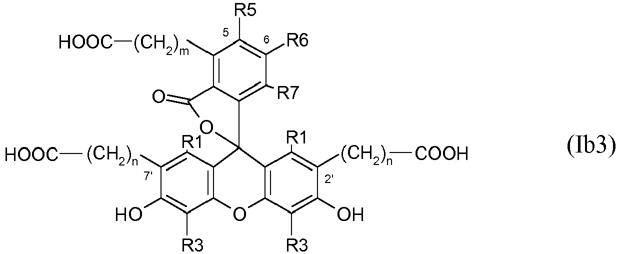 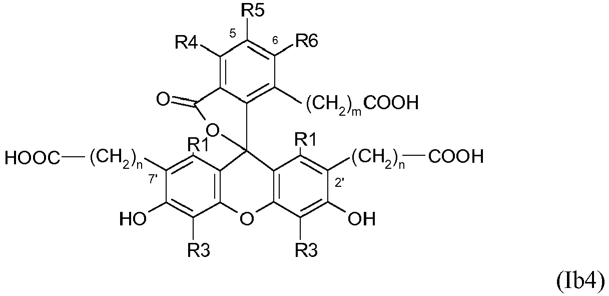 dans lesquelles m, n et R1, R3, R4, R5, R6, R7 différents de -A-COOH, sont tels que définis précédemment pour le composé de formule(Ib).

L'invention décrit aussi les composés répondant à la formule (Ic) : 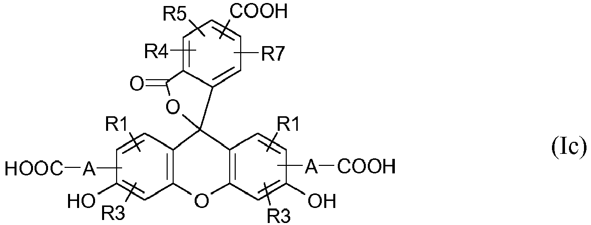 dans laquelle A et R1, R3, R4, R5, R7 différents de -A-COOH, sont tels que définis précédemment pour le composé de formule (I).

Avantageusement les composés décrits sont choisis parmi les isomères de position répondant à l'une des formules (Ic1) à (Ic4) qui suivent : 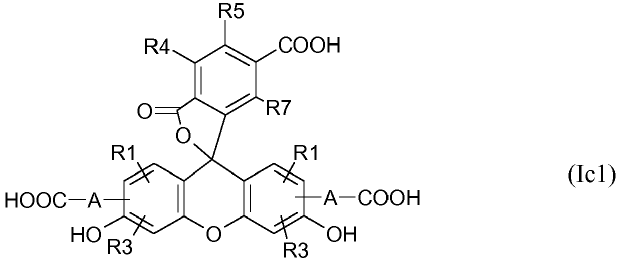 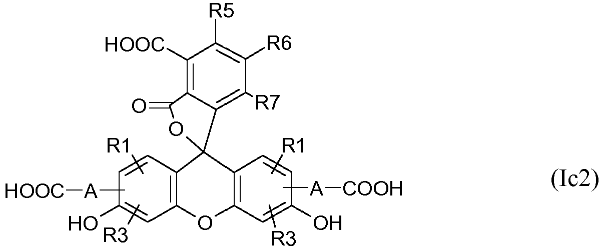 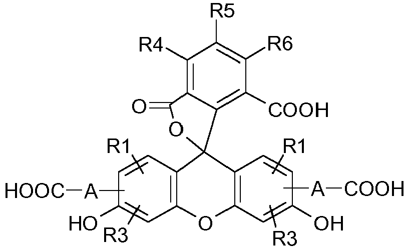 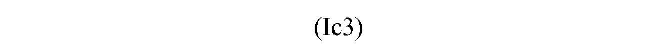 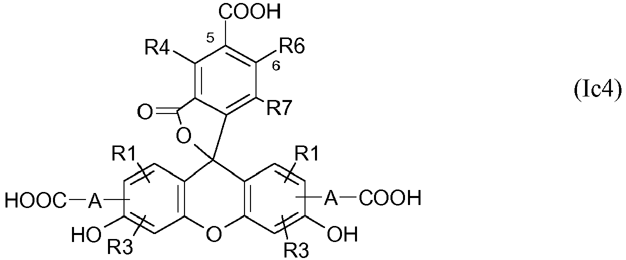 dans lesquelles A et R1, R3, R4, R5, R6, R7 sont tels que définis précédemment pour le composé de formule(Ic).

Encore plus avantageusement les composés décrits sont ceux pour lesquels A représente un groupement choisi dans le groupe comprenant le groupe -(CH₂)₂-et le groupe -CH=CH-.

Très avantageusement les composés décrits sont choisis dans le groupe comprenant les isomères de formule (Id1) à (Id8) qui suivent : 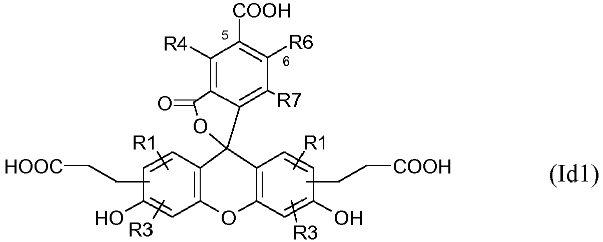 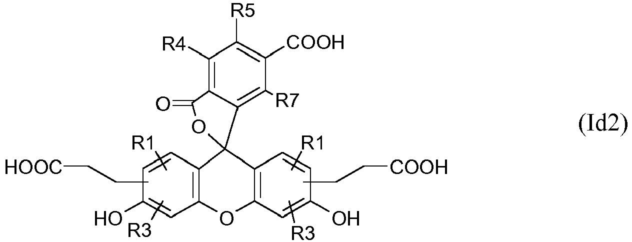 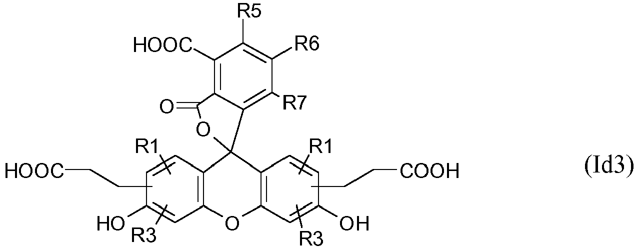 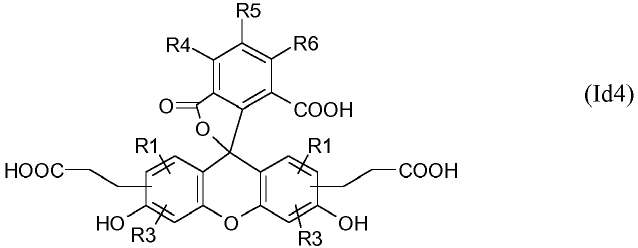 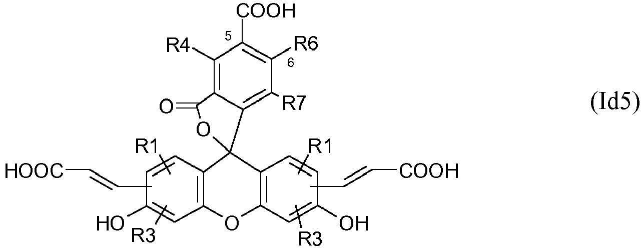 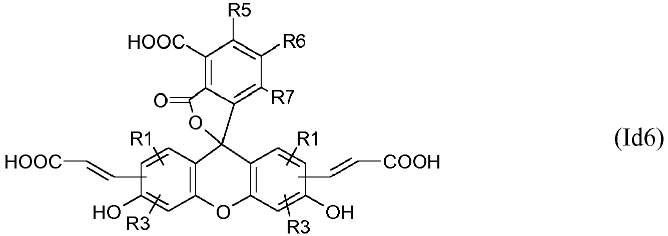 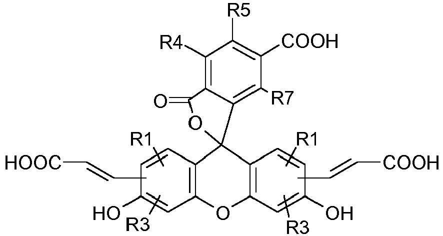 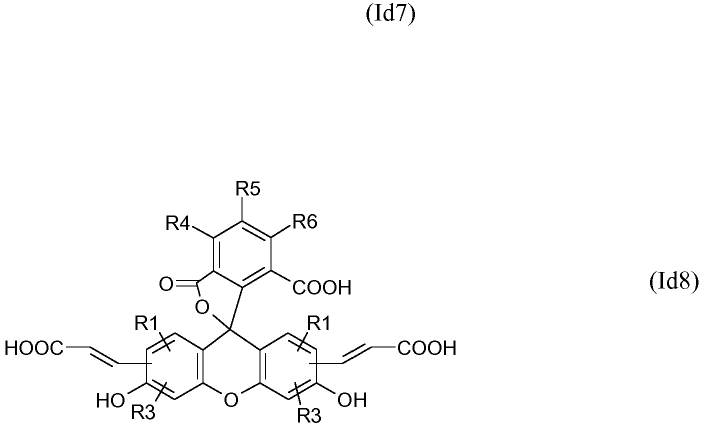 dans lesquelles R1, R3, R4, R5, R6, R7 différents de -A-COOH sont tels que définis précédemment pour le composé de formule(I).

De manière particulièrement avantageuse, les composés décrits sont choisis dans le groupe comprenant :
la 2',7'-bis(2-carboxyéthyl)-5-carboxyfluorescéine de formule : 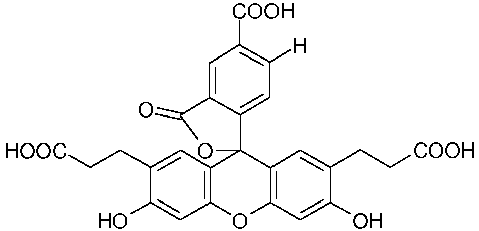
la 2',7'-bis(2-carboxyéthyl)-6-carboxyfluorescéine de formule : 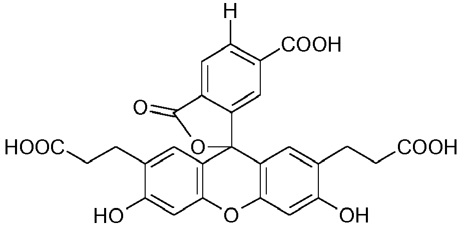
la 2',7'-Bis(2-carboxy-1,2-déhydroéthyl)-5-carboxy-fluorescéine de formule : 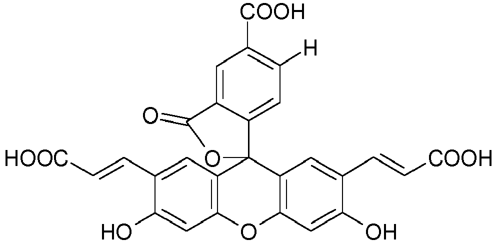
et la 2',7'-Bis(2-carboxy-1,2-déhydroéthyl)-6-carboxy-fluorescéine de formule :

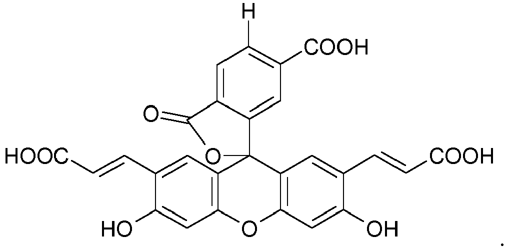

L'invention décrit en particulier des composés pour lesquels au moins un et préférentiellement au moins deux des R1, R2, R3 et R7, représentent indépendamment l'un de l'autre un groupement -A-COOH identique ou différent où A représente un groupement -(CH₂)ₙ-, n étant de préférence égal à 2 ou 3.

Alternativement, la présente invention décrit des composés de pureté isomérique égale à au moins 95%, avantageusement égale à au moins 98%, encore plus avantageusement égale à au moins 99% répondant à la formule (I), à l'exception de la 2',7'-bis(2-carboxyéthyl)-5-carboxyfluorescéine et de la 2',7'-bis(2-carboxyéthyl)-6-carboxyfluorescéine, de la 2',7'-bis(2-carboxypropyl)-5-carboxyfluorescéine, de la 2',7'-bis(2-carboxypropyl)-6-carboxyfluorescéine.

De préférence, la présente invention décrit des composés de pureté isomérique égale à au moins 95%, avantageusement égale à au moins 98%, encore plus avantageusement égale à au moins 99% répondant à la formule (I) telle que définie ci-dessus, dans laquelle, si R5 et/ou R6 représente un groupement carboxy, et si R2 représente un groupement -A-COOH où -A- est un groupe -(CH₂)ₙ-, alors n est supérieur à 3, soit compris entre 4 et 30.

Encore préférentiellement, la présente invention décrit des composés de pureté isomérique égale à au moins 95%, avantageusement égale à au moins 98%, encore plus avantageusement égale à au moins 99% répondant à la formule (I) telle que définie ci-dessus, dans laquelle, si R5 et/ou R6 représente un groupement carboxy, alors le groupement A de R2 est différent de -(CH₂)ₙ-.

De façon davantage préférée, la présente invention décrit des composés de pureté isomérique de position égale à au moins 95%, avantageusement égale à au moins 98%, encore plus avantageusement égale à au moins 99% répondant à la formule (I) telle que définie ci-dessus, dans laquelle, si R5 ou R6 représente un groupement carboxy, alors deux au moins des R4, R5, R6 et R7 ou au moins les deux groupements R1 ou au moins les deux groupements R3 représentent indépendamment l'un de l'autre un groupement -A-COOH identique ou différent ou un de ses sels alcalins, deux au moins des groupements A n'étant pas une simple liaison, où A est tel que défini précédemment ;
- R2 et tous les autres R1, R3, R4, R5, R6 et R7, identiques ou différents les uns des autres, sont choisis indépendamment les uns des autres, dans le groupe comprenant:
   ∘ l'hydrogène,
   ∘ les atomes d'halogène,
   ∘ le groupe hydroxy,
   ∘ le groupe amino,
   ∘ le groupe phosphate,
   ∘ le groupe nitro,
   ∘ le groupe sulfonate,
   ∘ les alkyles en C₁ à C₃₀, éventuellement ramifiés et/ou pouvant comprendre une ou plusieurs double(s) ou triple(s) liaison(s), éventuellement substitués par un ou plusieurs substituants choisis parmi halogéno, hydroxy, nitro, amino, sulfo, -CONHR et-CONR11R12 avec R, R11 et R12 représentant chacun indépendamment un groupe alkyle en C₁ à C₃₀,
   ∘ les cycloalkyle en C₃ à C₁₂,
   ∘ les alkyloxy en C₁ à C₃₀,
   ∘ les aryles éventuellement substitués par un ou plusieurs groupes choisis parmi les groupements halogèno, hydroxyles,
   ∘ les aryloxy,
   ∘ les aryl(alkyle en C₁ à C₃₀),
   ∘ les bases puriques ou pyrimidiques,
   ∘ les oses,
   ∘ les oligosides,
   ∘ les polyosides,
   ∘ les polypeptides,
   ∘ les protéines,
   ∘ les phospholipides,
et les esters correspondants, notamment comme pro-médicaments.

L'invention décrit également les esters des composés de formule (I) susceptibles d'être obtenus par estérification spécifique des fonctions acides carboxyliques non aromatiques de ce composé de formule (I) avec un composé de formule (III) :

R8-OH (III)

dans laquelle
- R8 est choisi dans le groupe comprenant :
   ∘ les groupes alkyles linéaires ou ramifiés en C₁ à C₃₀ et/ou pouvant comprendre une ou plusieurs double(s) ou triple(s) liaison(s), éventuellement substitués par un ou plusieurs substituants choisis dans le groupe comprenant les groupes halogéno, hydroxy, nitro, amino, et -COOR où R est un groupe alkyle en C₁ à C₃₀,
   ∘ cycloalkyles en C₃ à C₁₂,
   ∘ alkyloxyalkylène linéaires ou ramifiés en C₁ à C₃₀
   ∘ aryles éventuellement substitués par un ou plusieurs groupes choisis parmi les groupes halogéno, hydroxy et ester,
   ∘ aryloxyalkylène en C₁ à C₃₀,
   ∘ aryl-alkyle en C₁ à C₃₀.

L'invention décrit également les esters spécifiques des composés de formule 1 choisis dans le groupe comprenant :
la 2',7'-bis(2-carbométhoxyéthyl)-5-carboxyfluorescéine ;
la 2',7'-bis(2-carbométhoxyéthyl)-6-carboxyfluorescéine;
la 2',7'-bis(2-carboxyméthoxy-1,2-déhydroéthyl)-5-carboxyfluorescéine ;
la 2',7'-bis(2-carboxyméthoxy-1,2-déhydroéthyl)-6-carboxyfluorescéine.

Les inventeurs ont découvert qu'il était possible d'obtenir des colorants fluorescents polycarboxyliques de pureté isomérique de position élevée avec un rendement satisfaisant en réalisant la condensation en deux étapes successives et en faisant intervenir à un stade approprié une estérification spécifique des fonctions acides carboxyliques (-COOH) qui ne sont pas liées directement au cycle aromatique, tout en laissant libres les groupes -COOH qui sont directement liés à un cycle aromatique du colorant.

Les inventeurs ont découvert que cette estérification spécifique permettait non seulement d'améliorer la spécificité et le rendement des étapes de condensation pour la synthèse du colorant mais surtout de permettre une séparation aisée des isomères de position de ce colorant puisque les isomères de ces esters spécifiques peuvent être séparés par lavage et recristallisation.

Ceci était particulièrement inattendu puisque les techniques de séparation de l'art antérieur sont inefficaces lorsqu'elles sont appliquées après estérification non spécifique du colorant polycarboxylique.

Par estérification non spécifique, on entend l'estérification de toutes les fonctions acides carboxyliques du colorant, qu'elles soient aromatiques, c'est à dire fixées directement sur les cycles ou qu'elles soient non aromatiques, c'est à dire fixées sur les cycles par au moins un carbone. Ainsi, les groupements -A-COOH lorsque A représente -(CH₂)ₙ où n=0 et les autres groupements -COOH directement fixés sur les cycles sont des fonctions carboxyliques aromatiques ; en revanche les autres groupements -A-COOH dans lesquels A est différent de -(CH₂)ₙ où n=0 sont des fonctions carboxyliques non aromatiques.

De même, il est rappelé que l'application directe des techniques de séparation de l'art antérieur sur les isomères du colorant polycarboxylique ne permet pas l'obtention d'une séparation de ses isomères.

Ainsi les inventeurs ont découvert que chaque isomère pur du colorant peut être isolé sous forme de son ester spécifique et qu'il peut être ensuite régénéré par saponification de cet ester.

Aussi l'invention a donc pour objet un procédé de préparation d'un composé de formule (Ic) de pureté isomérique de position égale à au moins 95%, avantageusement égale à au moins 98%, encore plus avantageusement égale à au moins 99%, choisis parmi :
la 2',7'-bis(2-carboxyéthyl)-5-carboxyfluorescéine de formule : 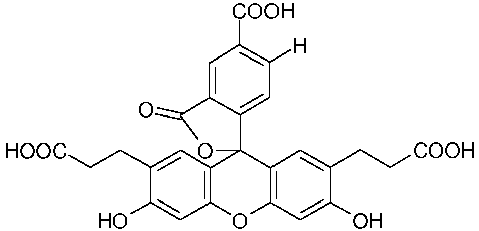
la 2',7'-bis(2-carboxyéthyl)-6-carboxyfluorescéine de formule : 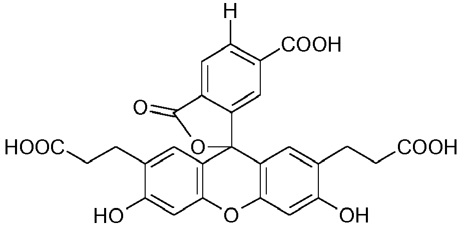
la 2',7'-Bis(2-carboxy-1,2-déhydroéthyl)-5-carboxy-fluorescéine de formule : 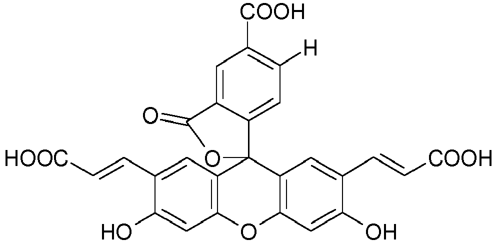
et la 2',7'-Bis(2-carboxy-1,2-déhydroéthyl)-6-carboxy-fluorescéine de formule : 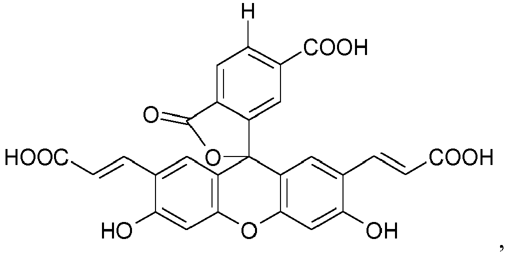
qui comprend une étape d'estérification spécifique des fonctions acides carboxyliques non aromatiques d'un composé répondant à la formule (Ic), ou d'un de ses intermédiaires de synthèse, ayant une pureté isomérique de position inférieure à 95% (i.e. étant constitué d'un mélange de plusieurs isomères), suivie d'une étape de séparation des esters obtenus, puis éventuellement d'une étape de saponification desdits esters.

Dans un mode avantageux de réalisation de l'invention, le procédé comprend en outre une étape de séparation des esters obtenus puis éventuellement une étape de saponification desdits esters.

D'autre part, et de façon inattendue, les inventeurs ont découvert qu'il était possible d'estérifier de façon spécifique les fonctions non aromatiques des colorants polycarboxyliques en les faisant réagir avec BF₃ dissous dans un alcool primaire ou secondaire à une température comprise entre 10°C et 40°C.

Avantageusement, la réaction d'estérification spécifique est réalisée avec un composé de formule (III)

R8-OH (III)

dans laquelle
- R8 est choisi dans le groupe comprenant :
   ∘ les groupes alkyles linéaires ou ramifiés en C₁ à C₃₀ et/ou pouvant comprendre une ou plusieurs double(s) ou triple(s) liaison(s), éventuellement substitués par un ou plusieurs substituants choisis dans le groupe comprenant les groupes halogéno, hydroxy, nitro, amino, et -COOR où R est un groupe alkyle en C₁ à C₃₀,
   ∘ cycloalkyles en C₃ à C₁₂,
   ∘ alkyloxyalkylène linéaires ou ramifiés en C₁ à C₃₀
   ∘ aryles éventuellement substitués par un ou plusieurs groupes choisis parmi les groupes halogéno, hydroxy et ester,
   ∘ aryloxyalkylène en C₁ à C₃₀,
   ∘ aryl-alkyle en C₁ à C₃₀.

Dans un premier mode de réalisation du procédé selon l'invention, on fait réagir un composé de formule (III)

R8-OH (III)

dans laquelle
- R8 est choisi dans le groupe comprenant :
   ∘ les groupes alkyles linéaires ou ramifiés en C₁ à C₃₀ et/ou pouvant comprendre une ou plusieurs double(s) ou triple(s) liaison(s), éventuellement substitués par un ou plusieurs substituants choisis dans le groupe comprenant les groupes halogéno, hydroxy, nitro, amino, et -COOR où R est un groupe alkyle en C₁ à C₃₀,
   ∘ cycloalkyles en C₃ à C₁₂,
   ∘ alkyloxyalkylène linéaires ou ramifiés en C₁ à C₃₀
   ∘ aryles éventuellement substitués par un ou plusieurs groupes choisis parmi les groupes halogéno, hydroxy et ester,
   ∘ aryloxyalkylène en C₁ à C₃₀,
   ∘ aryl-alkyle en C₁ à C₃₀,
avec un composé de formule (II) 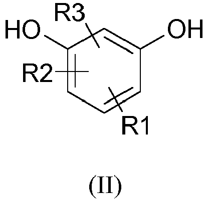 dans laquelle
- R2 représente un groupe -A-COOH avec A représentant un groupement-(CH₂)₂- ou -CH=CH-,
   ∘ et R1 et R3 représentent H,
   pour réaliser une estérification spécifique du ou des groupements carboxyliques non aromatiques et obtenir un composé de formule (IV) 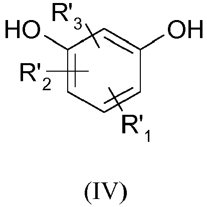 R'2 représente un groupe -A-COOR8 avec A représentant un groupement-(CH₂)₂- ou -CH=CH-,
   ∘ et R1 et R3 représentent H.

Dans un mode de réalisation avantageux du procédé selon l'invention, l'étape d'estérification spécifique est réalisée en présence d'un acide de Lewis ou d'un acide de Bronsted en solution dans R8-OH à une température comprise entre 10 °C et le point d'ébullition de R8-OH pendant une durée comprise entre 2 heures à 24 heures.

Les acides de Lewis avantageusement utilisés sont choisis dans le groupe comprenant BF₃, AlCl₃ et ZnCl₂ et les acides de Bronsted sont avantageusement choisis parmi l'acide toluène sulfonique et l'acide méthane sulfonique.

De manière encore plus avantageuse la réaction est conduite en présence de BF₃ dissous dans l'alcool R8-OH lui-même avantageusement choisi dans le groupe comprenant le méthanol, l'éthanol et l'isopropanol.

Dans un autre mode de réalisation du procédé selon l'invention, on fait réagir un composé de formule (III)

R8-OH (III)

tel que défini précédemment avec un composé de formule (V) 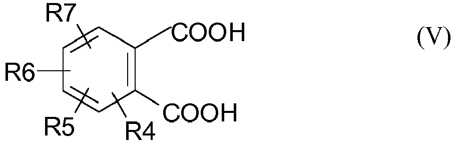 dans laquelle R6 est COOH et dans laquelle R4, R5, R7 représentent l'hydrogène,
pour réaliser une estérification spécifique des groupements carboxyliques non aromatiques et obtenir un composé de formule (VI) 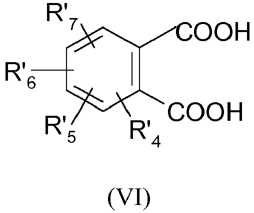 dans laquelle R'6 est COOH et et dans laquelle R'4, R'5, R'7 représentent l'hydrogène.

Dans un mode de réalisation avantageux de l'invention, l'étape d'estérification spécifique est réalisée en présence d'un acide de Lewis ou d'un acide de Bronsted dans les conditions décrites précédemment.

Dans un autre mode de réalisation de l'invention, le procédé comprend :
- une première étape de condensation d'un composé de formule (IV) tel que défini précédemment, avec un composé de formule (VI) tel que défini précédemment, ou avec son anhydride de formule (VI') 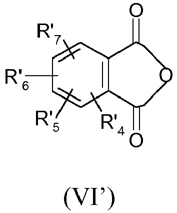 dans laquelle les groupes R'4, R'5, R'6, R'7 sont tels que définis précédemment pour le composé de formule (VI).
   pour obtenir un intermédiaire de formule (VII) 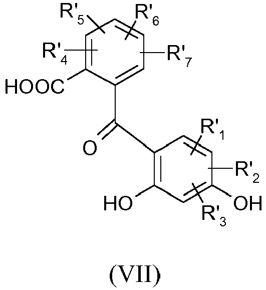 dans laquelle les groupes R'1, R'2, R'3, R'4, R'5, R'6 et R'7 sont tel que défini précédemment.
- une deuxième étape de condensation du composé de formule (VII) avec un composé de formule (IV) pour obtenir un composé de formule (VIII): 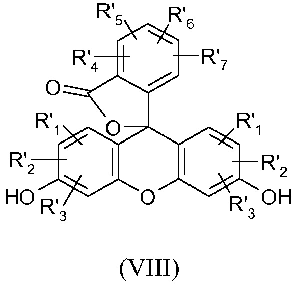 dans laquelle les groupes R'1, R'2, R'3, R'4, R'5, R'6, R'7 sont tels que définis précédemment pour les composés de formule (VII) et (IV).
- une étape de séparation des isomères de position du composé de formule (VIII) par lavage et/ou recristallisation, dans un alcool primaire ou secondaire, puis
- une étape de saponification des fonctions avec une base alcaline afin de générer le colorant polycarboxylique sous forme d'un seul isomère de position.

Avantageusement la première étape de condensation est réalisée dans les conditions d'une réaction de Friedel et Crafts, notamment en présence d'un acide de Lewis comme AlCl₃ ou ZnCl₂ en milieu nitrobenzène ou nitrométhane à une température comprise entre 15 °C et 110 °C pendant 5 à 72 heures.

Avantageusement la seconde étape de condensation est réalisée en présence d'un acide de Bronsted choisi dans le groupe comprenant l'acide sulfurique, l'acide polyphosphorique, l'acide phosphorique, les acides arylsulfoniques ou alkylsulfoniques, supportés ou non sur un polymère et leurs mélanges, éventuellement dans un solvant aprotique dipolaire, choisi dans le groupe comprenant la N-méthylpyrrolidone, le N,N-diméthylformamide, le formamide sous pression, l'acétonitrile sous pression et leurs mélanges. De manière très avantageuse, le solvant aprotique est la N-méthylpyrrolidone.

Dans un mode de réalisation particulièrement avantageux de réalisation, cette seconde étape de condensation est réalisée dans l'acide phosphorique qui sert à la fois de solvant et de catalyseur avec un diluant aprotique dipolaire tel que défini précédemment, de préférence dans la N-méthylpyrrolidone.

Avantageusement l'étape de séparation des isomères de position est réalisée par lavage et/ou recristallisation, dans un alcool primaire ou secondaire avantageusement choisi dans le groupe comprenant l'éthanol ou l'isopropanol.

Avantageusement l'étape de saponification des fonctions esters est réalisée avec une base alcaline, avantageusement choisie dans le groupe comprenant la soude, la potasse et le carbonate de sodium, à une température de 20°C à 90°C pendant une durée de 10 minutes à 4 heures selon la température et la base utilisée. Cette réaction de saponification permet de régénérer le colorant polycarboxylique sous forme d'un seul isomère de position de formule (I).

Dans un autre mode de réalisation de l'invention, le procédé comprend :
- une étape de condensation du composé de formule (II) avec un composé de formule (V) pour obtenir un intermédiaire de formule (IX): 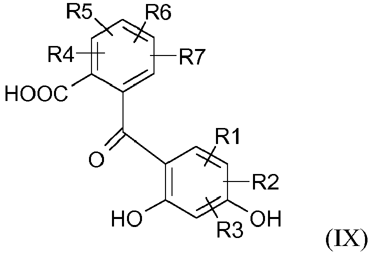 dans laquelle les groupes R1, R2, R3, R4, R5, R6, R7 sont tels que définis précédemment pour les composés de formules (II) et (V).
- une étape d'estérification spécifique du composé de formule (IX) avec un alcool de formule R8-OH (III)
   pour réaliser une estérification sélective des fonctions acides carboxyliques non aromatiques et obtenir le composé intermédiaire de formule (VII) : 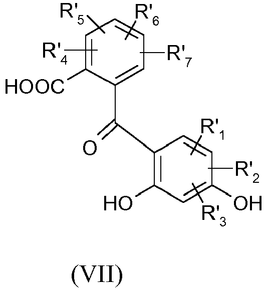 dans laquelle R'1, R'2, R'3, R'4, R'5, R'6 et R'7 sont tels que définis précédemment,
- une étape de condensation du composé de formule (VII) avec un composé de formule (IV) pour obtenir le composé de formule (VIII): 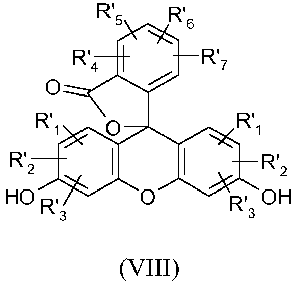 dans laquelle les groupes R'1, R'2, R'3, R'4, R'5, R'6, R'7 sont tels que définis précédemment.
- une étape de séparation des isomères de position du composé de formule (VIII) par lavage et/ou recristallisation dans un alcool primaire ou secondaire puis
- une étape de saponification des fonctions esters avec une base alcaline pour générer le colorant polycarboxylique sous forme d'un seul isomère de position de formule (I).

Dans un mode de réalisation avantageux de ce procédé, la première étape de condensation est réalisée en présence d'un acide de Lewis en milieu nitrobenzène ou nitrométhane à une température comprise entre 15 °C et 110 °C pendant 5 à 72 heures, la seconde étape de condensation est réalisée en présence d'un acide de Bronsted choisi dans le groupe comprenant l'acide sulfurique, l'acide polyphosphorique, l'acide phosphorique, les acides arylsulfoniques ou alkylsulfoniques, supportés ou non sur un polymère et leurs mélanges, éventuellement dans un solvant aprotique dipolaire, choisi dans le groupe comprenant la N-méthylpyrrolidone, le N,N-diméthylformamide, le formamide sous pression, l'acétonitrile sous pression et leurs mélanges, l'étape de séparation et de saponification étant réalisées dans les conditions décrites précédemment. Dans un mode de réalisation avantageux de l'invention on utilise, pour la deuxième étape de condensation, l'acide phosphorique dans un solvant aprotique dipolaire, de préférence la N-méthylpyrrolidone.

Dans un mode de réalisation avantageux de l'invention, utilise le BF₃ dissous dans un alcool primaire ou secondaire à une température comprise entre 10°C et 40°C pour réaliser l'estérification spécifique des fonctions carboxyliques non aromatiques.

Dans un autre mode de réalisation de l'invention, le procédé comprend :
- une étape de condensation du composé de formule (II) avec un composé de formule (V) pour obtenir un intermédiaire de formule (IX): 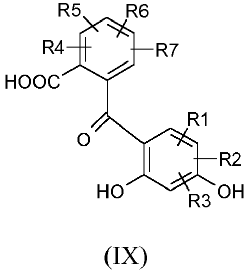 dans laquelle les groupes R1, R2, R3, R4, R5, R6, R7 sont tels que définis précédemment.
- une étape de purification de l'intermédiaire de formule (IX) par lavage et/ou recristallisation.
- une étape de condensation du composé intermédiaire de formule (IX) avec un composé de formule (II) pour obtenir le composé de formule : 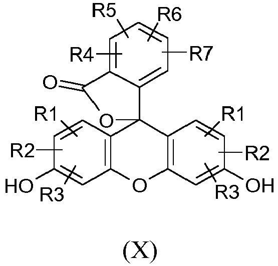 dans laquelle les groupes R1, R2, R3, R4, R5, R6, R7 sont tels que définis précédemment pour le composé de formule (I).
- une étape d'estérification spécifique du composé de formule (X) avec un alcool de formule R8-OH (III)
   pour réaliser une estérification sélective des fonctions acides carboxyliques non aromatiques et obtenir le composé intermédiaire de formule (VIII) : 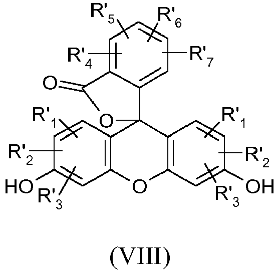 dans laquelle les groupes R'1, R'2, R'3, R'4, R'5, R'6, R'7 sont tels que définis précédemment.
- une étape de séparation des isomères de position du composé de formule (VIII) par lavage et/ou recristallisation,
- une étape de saponification des fonctions esters permettant de régénérer le colorant polycarboxylique sous forme d'un seul isomère de position de formule (I).

Dans un mode de réalisation avantageux de ce procédé, la première étape de condensation est réalisée en présence d'un acide de Lewis en milieu nitrobenzène ou nitrométhane à une température comprise entre 15 °C et 110 °C pendant 5 à 72 heures, la seconde étape de condensation est réalisée en présence d'un acide de Bronsted choisi dans le groupe comprenant l'acide sulfurique, l'acide polyphosphorique, l'acide phosphorique, les acides arylsulfoniques ou alkylsulfoniques, supportés ou non sur un polymère et leurs mélanges, éventuellement dans un solvant aprotique dipolaire, choisi dans le groupe comprenant la N-méthylpyrrolidone, le N,N-diméthylformamide, le formamide sous pression, l'acétonitrile sous pression et leurs mélanges, l'étape de séparation et de saponification étant réalisées dans les conditions décrites précédemment. Dans un mode de réalisation avantageux de l'invention on utilise, pour la deuxième étape de condensation, l'acide phosphorique dans un solvant aprotique dipolaire, de préférence la N-méthylpyrrolidone.

Dans un mode de réalisation avantageux de l'invention, utilise le BF₃ dissous dans un alcool primaire ou secondaire à une température comprise entre 10°C et 40°C pour réaliser l'estérification spécifique des fonctions carboxyliques non aromatiques.

Dans un autre mode de réalisation de l'invention, le procédé comprend :
- la préparation d'un composé de formule (VII) : 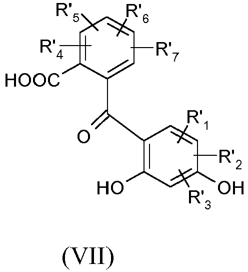 dans laquelle les groupes R'1, R'2, R'3, R'4, R'5, R'6, R'7 sont tels que définis précédemment.
- la séparation des isomères de position de l'intermédiaire (VII) par recristallisation,
- une étape de condensation de l'isomère ainsi isolé avec le composé de formule (IV) pour donner le composé de formule (VIII)
- une étape de purification des isomères de position du composé de formule (VIII) ainsi obtenu par lavage et/ou recristallisation,
- une étape de saponification des fonctions esters permettant de régénérer le colorant polycarboxylique sous forme d'un seul isomère de position de formule (I).

Dans un mode de réalisation avantageux de ce procédé, la première étape de condensation est réalisée en présence d'un acide de Lewis en milieu nitrobenzène ou nitrométhane à une température comprise entre 15 °C et 110 °C pendant 5 à 72 heures, la seconde étape de condensation est réalisée en présence d'un acide de Bronsted choisi dans le groupe comprenant l'acide sulfurique, l'acide polyphosphorique, l'acide phosphorique, les acides arylsulfoniques ou alkylsulfoniques, supportés ou non sur un polymère et leurs mélanges, éventuellement dans un solvant aprotique dipolaire, choisi dans le groupe comprenant la N-méthylpyrrolidone, le N,N-diméthylformamide, le formamide sous pression, l'acétonitrile sous pression et leurs mélanges, l'étape de séparation et de saponification étant réalisées dans les conditions décrites précédemment. Dans un mode de réalisation avantageux de l'invention on utilise, pour la deuxième étape de condensation, l'acide phosphorique dans un solvant aprotique dipolaire, de préférence la N-méthylpyrrolidone.

Dans un mode de réalisation avantageux de l'invention, utilise le BF₃ dissous dans un alcool primaire ou secondaire à une température comprise entre 10°C et 40°C pour réaliser l'estérification spécifique des fonctions carboxyliques non aromatiques.

Au cours de chacun des procédés précédemment décrits, le colorant polycarboxylique de pureté isomérique élevée peut éventuellement, en utilisant les techniques connues de l'art antérieur au niveau de l'étape la plus appropriée de la synthèse, être couplé au niveau de l'un ou plusieurs de ses groupes R1, R2, R3, R4, R5, R6, R7 à l'un des groupements similaires ou différents suivants :
∘ les oses,
∘ les oligosides,
∘ les polyosides,
∘ les polypeptides,
∘ les protéines,
∘ les phospholipides.

La présente invention décrit également l'utilisation d'un composé de formule (I) comme colorant fluorescent.

La présente invention décrit également des compositions de colorants fluorescents dans lesquelles le colorant fluorescent est un composé de formule (I).

La présente invention décrit également l'utilisation de compositions de colorants polycarboxyliques fluorescents selon l'invention ainsi que des colorants polycarboxyliques fluorescents obtenus à l'aide des procédés selon l'invention comme sondes de pH *in vivo* chez l'homme mais également *in vitro*.

En effet les colorants fluorescents polycarboxyliques décrits dans la présente demande ont la propriété, lorsqu'ils sont soumis à une ou plusieurs longueurs d'onde d'excitation, de fluorescer avec une intensité variable en fonction du pH du milieu. Ainsi, ces colorants polycarboxyliques permettent la mesure du pH par quantification de l'intensité de leur fluorescence à une ou plusieurs longueurs d'ondes d'excitation données. Le nombre de fonctions acides carboxyliques présentes sur ces colorants ayant une incidence notable sur leur sensibilité dans la gamme de pH concernée par la mesure.

Les colorants de l'invention décrite dans la présente demande permettent la réalisation de nouvelles techniques d'imagerie médicale en fluorescence, notamment l'exploration de la physiologie tissulaire *in vivo* chez l'homme mais également *in vitro*. Ce nouveau mode d'investigation médicale a pour objet l'imagerie *in vivo, mais également in vitro* et en temps réel des variations d'état physiologique au niveau tissulaire. Cette nouvelle technique d'imagerie conduit à une évaluation de la souffrance tissulaire au niveau d'une partie précise de l'organe, ce qui confère à ces colorants un très grand intérêt, notamment en toxicologie, en pharmacologie et en médecine où elles représentent un potentiel intéressant pour l'industrie pharmaceutique, en particulier pour le diagnostic médical et la réalisation d'études pharmacologiques *in vivo,* mais surtout *in vitro*.

Du fait que les compositions de colorants fluorescents polycarboxyliques selon l'invention contiennent au moins 95 %, de préférence au moins 98 %, et plus préférentiellement encore au moins 99 % en poids d'un seul isomère de position relativement au poids de l'ensemble des isomères, elles sont particulièrement utiles pour la détermination du pH tissulaire *in vivo* et *in vitro* chez l'homme.

Ces sondes de pH trouvent leur application par exemple dans le domaine du diagnostic, dans le domaine de la toxicologie, de la pharmacologie et en médecine, par exemple pour la réalisation de nouvelles techniques d'imagerie médicale en fluorescence, notamment l'exploration de la physiologie tissulaire *in vivo* chez l'homme. L'objectif étant d'imager en temps réel, des variations d'état physiologique d'un organe au niveau tissulaire, et notamment d'évaluer la souffrance tissulaire.

L'invention décrit également l'utilisation des composés selon l'invention dans le domaine du diagnostic médical.

L'invention décrit également l'utilisation des compositions de colorants fluorescents polycarboxyliques selon l'invention en imagerie médicale fonctionnelle par cartographie du pH tissulaire.

La 2',7'-Bis(2-carboxyéthyl)-5-carboxyfluorescéine et la 2',7'-Bis(2-carboxyéthyl)-6-carboxyfluorescéine sont particulièrement utiles comme sondes de pH pour la réalisation d'endoscopies digestives et pour la réalisation d'angiographies en fluorescence de la rétine car elle permet ainsi d'obtenir une cartographie de pH tissulaire de grande valeur diagnostique et pronostique pour la prise en charge thérapeutique du sepsis et pour la mise en évidence précoce des maladies micro vasculaire de l'oeil, telles que la dégénérescence maculaire liée à l'âge.

Les exemples 1 à 6 qui suivent illustrent la présente invention, sans en limiter en aucune façon la portée.

### EXEMPLE 1: Préparation de la 2',7'-Bis(2-carboxyéthyl)-5-carboxyfluorescéine)

### 1.1. Préparation de la 2',7'-Bis(2-carboxyéthyl)-5(6)-carboxyfluorescéine sous forme d'un mélange d'isomères de position

10 g (0,055 mol) d'acide 3-(2,4-dihydroxyphényl) propionique (ou acide 2,4-dihydroxyhydrocinnamique) sont condensés avec 5,3 g (0,028 mol) d'anhydride trimellitique en présence de 10 mL d'acide phosphorique dans 10 mL de N-méthylpyrolidone. Le mélange est porté à 190°C pendant 10 heures. Le milieu réactionnel devient rouge-brun foncé. Après refroidissement à 120°C, 120 mL de solution d'hydroxyde de sodium à 20% (m/m) sont lentement ajoutés. Le milieu réactionnel est maintenu sous agitation à 60°C pendant 1 heure avant d'être refroidi à température ambiante. Après acidification à pH 3, le produit brut est extrait avec un mélange acétate d'éthyle/éthanol (75/25 v/v). La phase organique est lavée avec une solution saturée de chlorure de sodium et séchée avec du sulfate de magnésium, puis évaporée à sec sous vide.
Masse du produit brut = 15 g
Rendement brut = quantitatif
RMN ¹H (DMSO D6): isomères 5 / 6 : ∼50/50 %

### 1.2. Préparation de la 2',7'-Bis(2-carbométhoxyéthyl)-5(6)-carboxyfluorescéine sous forme d'un mélange d'isomères de position

14 g de 2',7'-Bis(2-carboxyéthyl)-5(6)-carboxyfluorescéine obtenue dans l'étape 1.1. (0,027 mol) sont solubilisés dans 40 mL de méthanol. A cette solution sont ajoutés 6 mL de complexe BF₃/2MeOH (0,055 mol). Après 5 heures d'agitation à température ambiante, 75 mL d'eau salée sont ajoutés puis 100 mL d'acétate d'éthyle. La phase organique est lavée avec une solution saturée de chlorure de sodium, séchée avec du sulfate de magnésium, puis évaporée à sec.
Masse du produit brut = 15,3 g
Rendement brut = quantitatif
RMN ¹H (DMSO D6): isomères 5 / 6 : ∼50/50 %

### 1.3. Préparation de la 2',7'-Bis(2-carbométhoxyéthyl)-5-carboxyfluorescéine

15,3 g (0,028 mol) de 2',7'-Bis(2-carbométhoxyéthyl)-5(6)-carboxyfluorescéine obtenue dans l'étape 1.2. sont solubilisés à chaud dans 10 ml d'éthanol absolu puis sont concentrés. Après refroidissement à 4°C pendant au moins 24 heures, la résine obtenue est délitée avec 30 mL d'isopropanol. Le précipité est filtré et réempaté à chaud dans 30 mL d'éthanol. Ensuite, il est de nouveau filtré et lavé avec du tert-butylméthyléther.

Le filtrat est conservé pour la préparation de la 2',7'-Bis(2-carbométhoxyéthyl)-6-carboxyfluorescéine.
Masse du produit obtenu : 2 g
Rendement isomérique = 27,2 %
Rendement global = 13,6 %
Pureté isomérique déterminée par RMN ¹H :
Appareillage : Spectromètre BRUKER 400 MHz
Solvant : DMSO D6
2',7'-Bis(2-carbométhoxyéthyl)-5-carboxyfluorescéine = 97%
2',7'-Bis(2-carbométhoxyéthyl)-6-carboxyfluorescéine = 3%
Structure de la 2',7'-bis (2-carbométhoxyéthyl)-5-carboxyfluorescéine

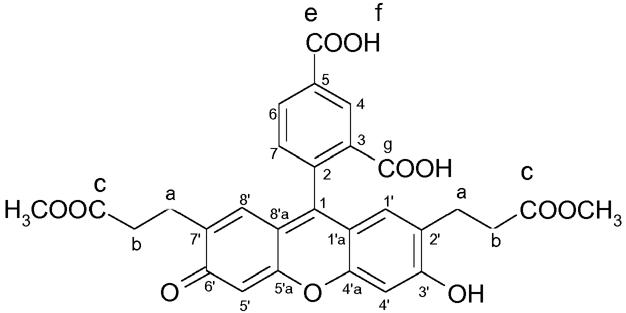

**RMN du Proton ¹H**

| **Proton** | **Signal** | **Intégration** | **Déplacement chimique δ (ppm)** |
|---|---|---|---|
| H_{b} | triplet | 4 | 2,39 |
| Hₐ | triplet | 4 | 2,61 |
| H_{d} | singulet | 6 | 3,41 |
| H_{1'} - H_{8'} | singulet | 2 | 6,43 |
| H_{4'}- H_{5'} | singulet | 2 | 6,70 |
| H₇ | doublet | 1 | 7,31 (J_{H7-H6}=2,0Hz) |
| H₆ | doublet | 1 | 8,29 (J_{H6-H7}= 2,0 Hz) |
| H₄ | singulet | 1 | 8,41 |
| OH | singulet | 2 | 10,28 |
| COOH | singulet aplati | Partiellement échangé | 13,54 |

**RMN du ¹³C (par JMOD)**

| **Carbone** | **Déplacement chimique δ (ppm)** | **Carbone** | **Déplacement chimique δ (ppm)** |
|---|---|---|---|
| Ca | 24,9 | C1'-C8' | 128,7 |
| Cb | 33,4 | C3 | 132,9 |
| Cd | 51,2 | C6 | 136,2 |
| C1 | 83,7 | C4'a-C5'a | 150,4 |
| C4'-C5' | 102,0 | C2 | 156,3 |
| C1'a-C8'a | 108,6 | C3' - C6' | 157,6 |
| C2'-C7' | 124,1 | Ce | 166,3 |
| C7 | 124,7 | Cg | 168,1 |
| C4 | 125,6 | Cc | 172,7 |
| C5 | 127,0 | | |

### 1.4. Préparation de la 2',7'-Bis(2-carboxyéthyl)-5-carboxyfluorescéine

2 g de 2',7'-Bis(2-carbométhoxyéthyl)-5-carboxyfluorescéine sont saponifiés dans 20 mL de solution d'hydroxyde de sodium 5 M à 80°C pendant 15 minutes. Après refroidissement et neutralisation avec 20 mL d'acide chlorhydrique 5 M, le 2',7'-Bis(2-carboxyéthyl)-5-carboxyfluorescéine est filtré et lavé à l'eau purifiée puis séché sous vide à 50°C.
Masse obtenue = 1,86 g (98%)
Pureté isomérique par RMN ¹H (DMSO D6):
2',7'-Bis(2-carboxyéthyl)-5-carboxyfluorescéine = 98,0 %
2',7'-Bis(2-carboxyéthyl)-6-carboxyfluorescéine = 2,0 %

### Structure de la 2',7'-bis (2-carboxyéthyl)-5-carboxyfluorescéine

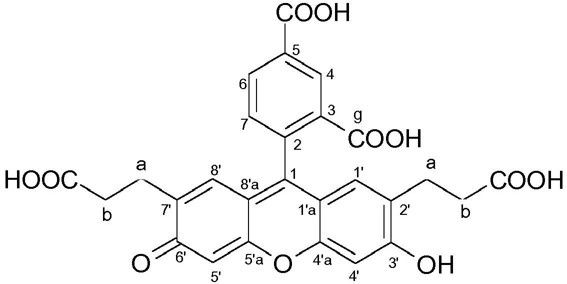

**RMN du Proton ¹H**

| **Proton** | **Signal** | **Intégration** | **Déplacement chimique δ (ppm)** |
|---|---|---|---|
| H_{b} | triplet | 4 | 2,39 |
| Hₐ | triplet | 4 | 2,61 |
| H_{1'}-H_{8'} | singulet | 2 | 6,43 |
| H_{4'}-H_{5'} | singulet | 2 | 6,70 |
| H₇ | doublet | 1 | 7,31 (J_{H7-H6}=2,0 Hz) |
| H₆ | doublet | 1 | 8,29 (J_{H6-H7}= 2,0 Hz) |
| H₄ | singulet | 1 | 8,41 |
| OH | singulet | partiellement échangé | 10,28 |
| COOH | singulet aplati | partiellement échangé | 13,54 |

### EXEMPLE 2: Préparation de la 2',7'-Bis(2-carboxyéthyl)-6-carboxyfluorescéine

### 2.1. Préparation de la 2',7'-Bis(2-carbométhoxyéthyl)-6-carboxyfluorescéine

Après évaporation du filtrat obtenu au cours de la préparation de la 2',7'-Bis(2-carbométhoxyéthyl)-5-carboxyfluorescéine (Exemple 1.3), le solide obtenu est porté à ébullition dans 10 mL d'isopropanol pendant 10 minutes. Après refroidissement à température ambiante, la 2',7'-Bis(2-carbométhoxyéthyl)-6-carboxyfluorescéine est filtrée et lavée avec de l'isopropanol préalablement refroidi à 4°C. L'opération est renouvelée. Puis le solide obtenu est lavé avec du terbutylméthyléther et séché sous vide.
Masse du produit obtenu : 1,1 g
Rendement isomérique = 15 %
Rendement global = 7,5 % Pureté isomérique par RMN ¹H (DMSO D6) :
2',7'-Bis(2-carbométhoxyéthyl)-5-carboxyfluorescéine = 4,0%
2',7'-Bis(2-carbométhoxyéthyl)-6-carboxyfluorescéine = 96,0%

### Structure de la 2',7'-bis (2-carbométhoxyéthyl)-6-carboxyfluorescéine

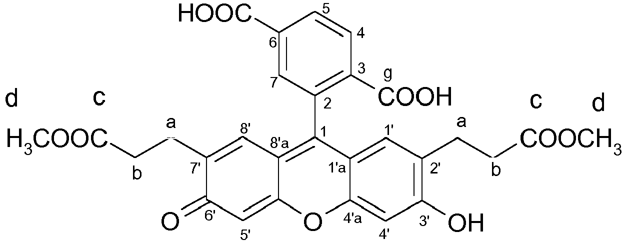

**RMN du Proton ¹H**

| **Proton** | **Signal** | **Intégration** | **Déplacement chimique δ (ppm)** |
|---|---|---|---|
| H_{b} | triplet | 4 | 2,39 |
| Hₐ | triplet | 4 | 2,61 |
| H_{d} | singulet | 6 | 3,40 |
| H_{1'}-H_{8'} | singulet | 2 | 6,40 |
| H_{4'}-H_{5'} | singulet | 2 | 6,71 |
| H₇ | singulet | 1 | 7,58 |
| H₄ | doublet | 1 | 8,12 (J_{H4-H5} = 2,0 Hz) |
| H₅ | doublet | 1 | 8,23 (J_{H5-H4}= 2,0 Hz) |
| OH | singulet | 2 | 10,29 |
| COOH | singulet aplati | partiellement échangé | 13,54 |

**RMN du ¹³C (par JMOD)**

| **Carbone** | **Déplacement chimique δ (ppm)** | **Carbone** | **Déplacement chimique δ (ppm)** |
|---|---|---|---|
| Ca | 24,9 | C6 | 129,7 |
| Cb | 33,3 | C5 | 131,0 |
| Cd | 51,1 | C3 | 137,4 |
| C1 | 83,7 | C4'a-C5'a | 150,4 |
| C4'-C5' | 102,0 | C2 | 152,9 |
| C1'a-C8'a | 108,6 | C3' - C6' | 157,5 |
| C2'-C7' | 124,0 | Ce | 166,3 |
| C7 | 124,6 | Cg | 168,2 |
| C4 | 125,4 | Cc | 172,7 |
| C1'-C8' | 128,6 | | |

### 2.2. Préparation de la 2',7'-Bis(2-carboxyéthyl)-6-carboxyfluorescéine

1,1 g de 2',7'-Bis(2-carbométhoxyéthyl)-6-carboxyfluorescéine est saponifié dans 11 mL de solution d'hydroxyde de sodium 5 M à 80°C pendant 15 minutes. Après refroidissement et neutralisation avec 11 mL d'acide chlorhydrique 5 M, le 2',7'-Bis(2-carboxyéthyl)-6-carboxyfluorescéine est filtré et lavé à l'eau purifiée puis séché sous vide à 50°C.
Masse obtenue = 1 g (rendement : 96 %)
Rendement isomérique = 14,4 %
%Rendement global : 7,2 %
Pureté isomérique par RMN ¹H (DMSO D6) :
2',7'-Bis(2-carboxyéthyl)-5-carboxyfluorescéine = 4,0 %
2',7'-Bis(2-carboxyéthyl)-6-carboxyfluorescéine = 96,0 %

### Structure de la 2',7'-bis (2-carboxyéthyl)-6-carboxyfluorescéine

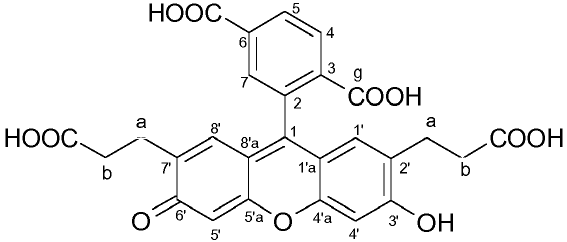

**RMN du Proton ¹H**

| **Proton** | **Signal** | **Intégration** | **Déplacement chimique δ (ppm)** |
|---|---|---|---|
| H_{b} | triplet | 4 | 2,39 |
| Hₐ | triplet | 4 | 2,61 |
| H_{1'}-H_{8'} | singulet | 2 | 6,40 |
| H_{4'}-H_{5'} | singulet | 2 | 6,71 |
| H₇ | singulet | 1 | 7,58 |
| H₄ | doublet | 1 | 8,12 (J_{H4-H5} = 2,0 Hz) |
| H₅ | doublet | 1 | 8,23 (J_{H5-H4}= 2,0 Hz) |
| OH | singulet | 2 | 10,29 |
| COOH | singulet aplati | partiellement échangé | 13,54 |

### EXEMPLE 3: Préparation de la 2',7'-Bis(2-carboxy-1,2-déhydroéthyl)-5-carboxyfluorescéine)

### 3.1. Préparation de la 2',7'-Bis(2-carboxy-1,2-déhydroéthyl)-5(6)-carboxyfluorescéine sous forme d'un mélange d'isomères de position

Porter à 190°C 5,3 g (0,028 mol) d'anhydride trimellitique solubilisés dans 10 mL d'acide phosphorique et 10 mL de N-méthylpyrolidone. Ajouter 10 g (0,055 mol) d'acide 2,4-dihydroxycinnamique. Le milieu réactionnel est maintenu à 190°C pendant 10 heures au cours duquel il devient rouge-brun foncé. Après refroidissement à 120°C, 120 mL de solution d'hydroxyde de sodium à 20% (m/m) sont lentement ajoutés. Le milieu réactionnel est maintenu sous agitation à 60°C pendant 1 heure avant d'être refroidi à température ambiante. Après acidification à pH 3, le produit brut est extrait avec un mélange acétate d'éthyle/éthanol (75/25 v/v). La phase organique est lavée avec une solution saturée de chlorure de sodium et séchée avec du sulfate de magnésium, puis évaporée à sec sous vide.
Masse du produit brut = 11,2 g
Rendement brut = 79,4%
RMN ¹H (DMSO D6): isomères 5 / 6 : ∼50/50 %

### 3.2. Préparation de la 2',7'-Bis(2-carbométhoxy-1,2-déhydroéthyl)-5(6)-carboxyfluorescéine sous forme d'un mélange d'isomères de position

10 g de 2',7'-Bis(2-carboxy-1,2-déhydroéthyl)-5(6)-carboxyfluorescéine obtenue dans l'étape 3.1. (0,019 mol) sont solubilisés dans 30 mL de méthanol. A cette solution sont ajoutés 4,1 mL de complexe BF₃/2MeOH (0,038 mol). Après 5 heures d'agitation à température ambiante, 50 mL d'eau salée sont ajoutés puis 70 mL d'acétate d'éthyle. La phase organique est lavée avec une solution saturée de chlorure de sodium, séchée avec du sulfate de magnésium, puis évaporée à sec.
Masse du produit brut = 8,4 g
Rendement brut = 80 %
RMN ¹H (DMSO D6): isomères 5 / 6 : ∼50/50

### 3.3. Préparation de la 2',7'-Bis(2-carbométhoxy-1,2-déhydroéthyl)-5-carboxyfluorescéine

8,4 g (0,015 mol) de 2',7'-Bis(2-carbométhoxy-1,2-déhydroéthyl)-5(6)-carboxyfluorescéine obtenue dans l'étape 3.2. sont solubilisés à chaud dans 10 ml d'éthanol absolu puis sont concentrés. Après refroidissement à 4°C pendant au moins 24 heures, la résine obtenue est délitée avec 30 mL d'isopropanol. Le précipité est filtré et réempaté à chaud dans 30 mL d'éthanol. Ensuite, il est de nouveau filtré et lavé avec du tert-butylméthyléther

Le filtrat est conservé pour la préparation de la 2',7'-Bis(2-carbométhoxy-1,2-déhydroéthyl)-6-carboxyfluorescéine.
Masse du produit obtenu : 1,3 g
Rendement isomérique = 24,8 % Rendement global = 14,4%
Pureté isomérique par RMN ¹H (DMSO D6) :
Isomère 5 = 95,8 % Isomère 6 = 4,2 %

### Structure de la 2',7'-bis (2-carbométhoxy-1,2-déhydroéthyl)-5-carboxyfluorescéine

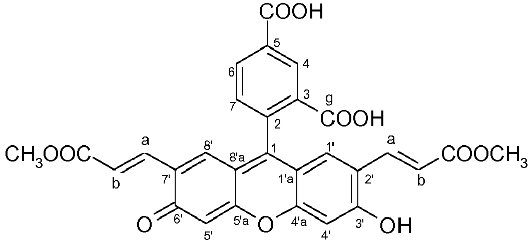

**RMN du Proton ¹H**

| **Proton** | **Signal** | **Intégration** | **Déplacement chimique δ (ppm)** |
|---|---|---|---|
| CH₃ | singulet | 6 | 6,43 |
| H_{b} | doublet | 2 | 6,24 |
| H_{4'}-H_{5'} | singulet | 2 | 6,81 |
| H_{1'}-H_{8'} | singulet | 2 | 7,41 |
| H₇ | doublet | 1 | 7,31 (J_{H7-H6}=2,0Hz) |
| Hₐ | doublet | 2 | 7,67 |
| H₆ | doublet | 1 | 8,29 (J_{H6-H7}= 2,0 Hz) |
| H₄ | singulet | 1 | 8,41 |
| OH | singulet | partiellement échangé | 10,17 |
| COOH | singulet aplati | partiellement échangé | 13,24 |

### 3.4. Préparation de la 2',7'-Bis(2-carboxy-1,2-déhydroéthyl)-5-carboxyfluorescéine

1,3 g de 2',7'-Bis(2-carbométhoxy-1,2-déhydroéthyl)-5-carboxyfluorescéine sont saponifiés dans 13 mL de solution d'hydroxyde de sodium 5M à 80°C pendant 15 minutes. Après refroidissement et neutralisation avec 13 mL d'acide chlorhydrique 5 M, la 2',7'-Bis(2-carboxy-1,2-déhydroéthyl)-5-carboxyfluorescéine est filtrée, lavée à l'eau purifiée puis séchée sous vide à 50°C.
Masse obtenue = 1,2 g (rendement : 97,5%)
Pureté isomérique par RMN ¹H (DMSO D6) :
Isomère 5 = 94,9%
Isomère 6 = 5,1%

### EXEMPLE 4: Préparation de la 2',7'-Bis(2-carboxy-1,2-déhydroéthyl)-6-carboxyfluorescéine

### 4.1. Préparation de la 2',7'-Bis(2-carbométhoxy-1,2-déhydroéthyl)-6-carboxyfluorescéine

Après évaporation du filtrat obtenu au cours de la préparation de la 2',7'-Bis(2-carbométhoxy-1,2-déhydroéthyl)-5-carboxyfluorescéine (Exemple 3.3), le solide obtenu est porté à ébullition dans 7 mL d'isopropanol pendant 10 minutes. Après refroidissement à température ambiante, la 2',7'-Bis(2-carbométhoxy-1,2-déhydroéthyl)-6-carboxyfluorescéine est filtrée et lavé avec de l'isopropanol préalablement refroidi à 4°C. L'opération est renouvelée. Puis le solide obtenu est lavé avec du terbutylméthyléther et séché sous vide.
Masse du produit obtenu : 0,9 g
Rendement : 8,5 %
Rendement isomérique = 17,1 %
RMN ¹H (DMSO D6):
Isomère 5 = 5,8 % Isomère 6 = 94,2 %

### Structure de la 2',7'-bis (2-carboxyméthoxy-1,2-déhydroéthyl)-6-carboxyfluorescéine

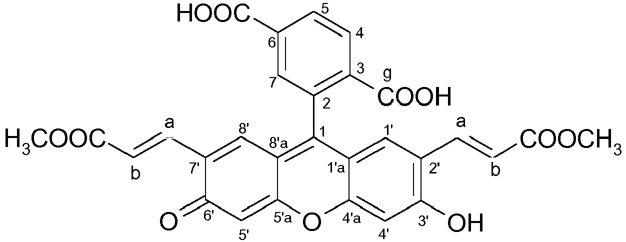

**RMN du Proton ¹H**

| **Proton** | **Signal** | **Intégration** | **Déplacement chimique δ (ppm)** |
|---|---|---|---|
| CH₃ | singulet | 6 | 3,43 |
| H_{b} | doublet | 2 | 6,24 |
| H_{4'}- H_{5'} | singulet | 2 | 6,82 |
| H_{1'}-H_{8'} | singulet | 2 | 7,41 |
| H₇ | doublet | 1 | 7,96 (J_{H7-H6}= 2,0 Hz) |
| Hₐ | doublet | 2 | 7,67 |
| H₆ | doublet | 1 | 8,30 (J_{H6-H7}= 2,0 Hz) |
| H₄ | singulet | 1 | 8,41 |
| OH | singulet | partiellement échangé | 10,25 |
| COOH | singulet aplati | partiellement échangé | 13,20 |

### 4.2. Préparation de la 2',7'-Bis(2-carboxy-1,2-déhydroéthyl)-6-carboxyfluorescéine

0,9 g de 2',7'-Bis(2-carbométhoxy-1,2-déhydroéthyl)-6-carboxyfluorescéine sont saponifiés dans 9 mL de solution d'hydroxyde de sodium 5 M à 80°C pendant 15 minutes. Après refroidissement et neutralisation avec 9 mL d'acide chlorhydrique 5 M, le 2',7'-Bis(2-carboxy-1,2-déhydroéthyl)-6-carboxyfluorescéine est filtré et lavé à l'eau purifiée puis séché sous vide à 50°C.
Masse obtenue = 0,82 g (rendement : 96,4 %)

Pureté isomérique par RMN ¹H (DMSO D6):
Isomère 5 = 5,4 % Isomère 6 = 94,6 %

### Exemple 5: préparation de l'acide 2-[5'-(2-carboxyéthyl)-2',4'-dihydroxybenzoyl]-5-carboxybenzoïque

### 5.1. Préparation de l'acide 2-[5'-(2-carboxyéthyl)-2',4'-dihydroxybenzoyl]-4(5)-carboxybenzoïque (mélange d'isomères de position)

On dissout 6 g d'acide 3-(2,4-dihydroxyphényl)-propionique (0,033 mol) dans 25 mL de nitrobenzène. On ajoute 9,3 g (0,033 mol) d'anhydride trimellitique benzyl ester puis 11 g (0,0825 mol) d'AlCl₃ en maintenant sous agitation pendant 48 heures à température ambiante. Le milieu réactionnel est extrait avec 1000 mL d'acétate d'éthyle. Le produit est séparé de la phase organique par l'ajout de 200 mL de soude 2N. La phase aqueuse est maintenue à 60°C pendant 30 minutes, permettant ainsi de saponifer l'ester benzylique. Après acidification à pH compris entre 1 et 3 avec de l'acide chlorhydrique 2N, l'acide 2-[5'-(2-carboxyéthyl)-2',4'-dihydroxybenzoyl]-4(5)-carboxybenzoïque est extrait par 500 mL l'acétate d'éthyle. Après lavage à l'eau purifiée, la phase organique est séchée avec du sulfate de magnésium, puis évaporée à sec sous vide.
Masse du mélange d'isomères obtenu = 9,2 g
Rendement = 74,5 %

### 5.2.Préparation de l'acide 2-[5'-(2-carbométhoxyéthyl)-2',4'-dihydroxybenzoyl]-4(5)-carboxybenzoïque (mélange d'isomères de position)

La méthylation spécifique de l'acide 2-[5'-(2-carboxyéthyl)-2',4'-dihydroxybenzoyl]-4(5)-carboxybenzoïque est réalisée selon la méthode de l'exemple 1.2. (rendement quantitatif).

### 5.3. Préparation de l'acide 2-[5'-(2-carbométhoxyéthyl)-2',4'-dihydroxybenzoyl]-4-carboxybenzoïque

Transférer le mélange d'isomères obtenu dans l'exemple 5.2. dans 50 mL de terbutylméthyléther. Porter à reflux pendant 30 minutes. Après refroidissement, le produit obtenu est filtré, lavé avec le même solvant puis recristallisé dans l'éthanol absolu.
Masse de l'isomère 4 = 3 g Rendement isomérique = 65,2 %
Pureté isomérique par RMN ¹H (DMSO D6):
Isomère 4 = 94,8% Isomère 5 = 5,2%
Le filtrat est conservé pour isoler l'isomère 5.

### Structure de l'acide 2-[5'-(2-carbométhoxyéthyl)-2',4'-dihydroxybenzoyl]-4-carboxybenzoïque

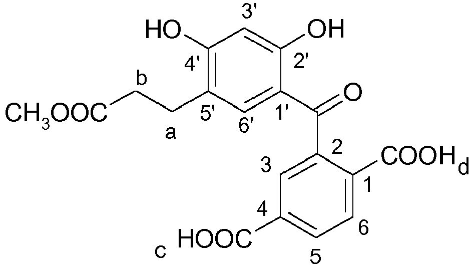

**RMN ¹H (DMSO D6**

| **Proton** | **Signal** | **Intégration** | **Déplacement chimique δ (ppm)** |
|---|---|---|---|
| Hb | triplet | 2 | 2,30 |
| Ha | triplet | 2 | 2,53 |
| CH₃ | singulet | 3 | 3,43 |
| H6' | singulet | 1 | 6,38 |
| H3' | singulet | 1 | 6,88 |
| H3 | singulet | 1 | 7,80 |
| H6 | doublet | 1 | 8,07 |
| H5 | doublet | 1 | 8,15 |
| OH | Singulet aplati | partiellement échangé | 10,9 |
| COOHd | Singulet aplati | partiellement échangé | 11,8 |
| COOHc | Singulet aplati | partiellement échangé | 13,1 |

### EXEMPLE 6: Préparation de la 2',7'-Bis(2-carboxyéthyl)-6-carboxyfluorescéine) à partir de l'acide 2-[5'-(2-carbométhoxyéthyl)-2',4'-dihydroxybenzoyl]-4-carboxybenzoïque

### 6.1. Préparation de la 2',7'-Bis(2-carboxyéthyl)-6-carboxyfluorescéine

1 g (0,0026 mol) d'acide 2-[5'-(2-carbométhoxy-éthyl)-2',4'-dihydroxybenzoyl]-4-carboxybenzoïque est condensé avec 0,47 g (0,026 mol) d'acide 3-(2,4-dihydroxyphényl)propionique (ou acide 2,4-dihydroxyhydrocinnamique) en présence de 1 mL d'acide phosphorique dans 1 mL de N-méthylpyrolidone. Les conditions de la réaction et le traitement du milieu réactionnel sont ceux décrits dans l'exemple 1.1.
Masse du produit brut = 1 g
Rendement brut = 71,9%
CLHP : isomère 6 = 98,1 %

Les puretés isomériques obtenues dans les exemples 1 à 6 peuvent encore être améliorées, par mise en oeuvre une seconde fois du procédé.

## Revendications

1. Procédé de préparation de composés de pureté isomérique de position égale à au moins 95% répondant à la formule (Ic) choisie dans le groupe comprenant :
la 2',7'-bis(2-carboxyéthyl)-5-carboxyfluorescéine de formule : 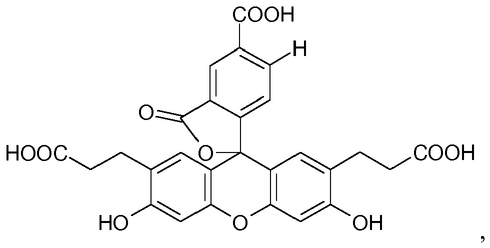
la 2',7'-bis(2-carboxyéthyl)-6-carboxyfluorescéine de formule : 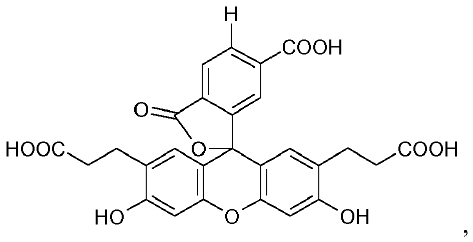
la 2',7'-Bis(2-carboxy-1,2-déhydroéthyl)-5-carboxy-fluorescéine de formule : 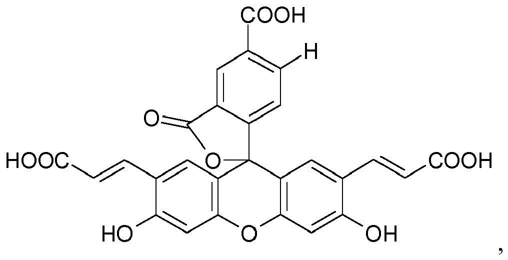 et
et la 2',7'-Bis(2-carboxy-1,2-déhydroéthyl)-6-carboxy-fluorescéine de formule : 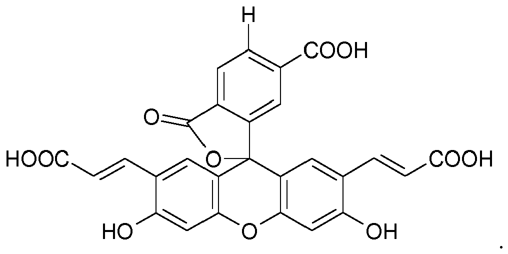 **caractérisé en ce que** ledit procédé comprend une étape d'estérification spécifique des fonctions acides carboxyliques non aromatiques d'un composé répondant à la formule (Ic) permettant d'obtenir un dérivé ester dudit composé de formule (Ic) dont les fonctions acides carboxyliques non aromatiques sont estérifiées avec une pureté isomérique de position inférieure à 95%, suivie d'une étape de séparation dudit dérivé ester, puis éventuellement d'une étape de saponification dudit dérivé ester.

2. Procédé de préparation selon la revendication 1, **caractérisé en ce qu'**il comprend :
- une étape de condensation du composé de formule (II) 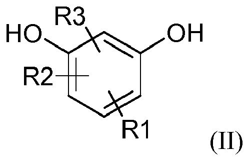
- dans laquelle R1 et R3 représentent H,
et R2 représente un groupe -A-COOH avec A représentant un groupement-(CH₂)₂- ou -CH=CH-,
avec un composé de formule (V) 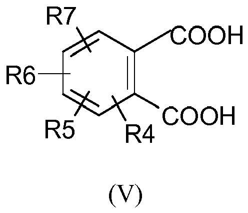
- dans laquelle R4, R5, R7 représentent l'hydrogène;
et R6 représente un groupe -COOH,
pour obtenir un intermédiaire de formule (IX): 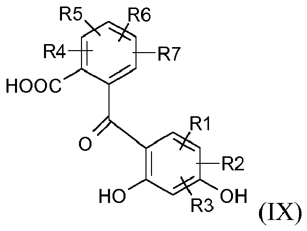 dans laquelle R1, R2, R3, R4, R5, R6, R7 sont tels que définis précédemment,
- une étape de purification de l'intermédiaire de formule (IX) par lavage et/ou recristallisation.
- une étape de condensation du composé intermédiaire de formule (IX) avec un composé de formule (II) pour obtenir le composé de formule : 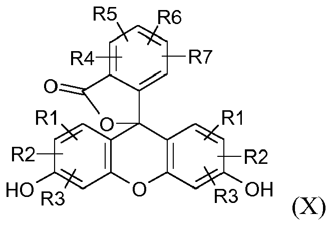
dans laquelle R1, R2, R3, R4, R5, R6 et R7 sont tels que définis précédemment ;
- une étape d'estérification spécifique du composé de formule (X) avec le composé de formule R8-OH (III) où R8 représente un groupe choisi dans le groupe comprenant :
∘ les groupes alkyles linéaires ou ramifiés en C₁ à C₃₀ et/ou pouvant comprendre une ou plusieurs double(s) ou triple(s) liaison(s), éventuellement substitués par un ou plusieurs substituants choisis dans le groupe comprenant les groupes halogéno, hydroxy, nitro, amino, et -COOR où R est un groupe alkyle en C₁ à C₃₀,
∘ cycloalkyles en C₃ à C₁₂,
∘ alkyloxyalkylène linéaires ou ramifiés en C₁ à C₃₀
∘ aryles éventuellement substitués par un ou plusieurs groupes choisis parmi les groupes halogéno, hydroxy et ester,
∘ aryloxyalkylène en C₁ à C₃₀,
∘ aryl-alkyle en C₁ à C₃₀,
pour réaliser une estérification sélective des fonctions acides carboxyliques non aromatiques et obtenir le composé intermédiaire de formule (VIII) : 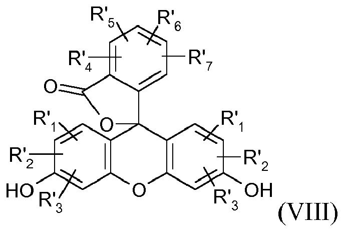
- dans laquelle R'1, R'3, R'4, R'5, R'6 et R'7 dans laquelle,
R'2 est un groupement -A-COO-R8, similaire ou différent, avec A et R8 tels que définis précédemment, l'un au moins des groupements A étant différent d'une simple liaison, et R' 1 et R'3 représentent un hydrogène,
et dans laquelle R'6 est un groupement -COOH et R'4, R'5et R'7 représentent un hydrogène,
- une étape de séparation des isomères de position du composé de formule (VIII) par lavage et/ou recristallisation,
- une étape de saponification des fonctions esters permettant de régénérer le composé de formule (Ic) tel que défini à la revendication 1 sous forme d'un seul isomère de position avec une pureté isomérique de position supérieure ou égale à 95%.

3. Procédé selon l'une quelconque des revendications 1 à 2, **caractérisé en ce que** l'étape d'estérification spécifique des fonctions acides carboxyliques non aromatiques est une réaction avec BF₃ dissous dans un alcool primaire ou secondaire, à une température de 10°C à 40°C.

4. Procédé selon la revendication 3, **caractérisé en ce que** l'alcool primaire ou secondaire est le méthanol, l'éthanol ou l'isopropanol.

## Patentansprüche

1. Zubereitungsverfahren von Verbindungen mit einer isomerenreinheit Position von mindestens 95 %, das der Formel (Ic) entspricht, die ausgewählt sind aus der Gruppe, umfassend:
Das 2', 7'-bis(2-Carboxyethyl)-5-Carboxyfluorescein der Formel: 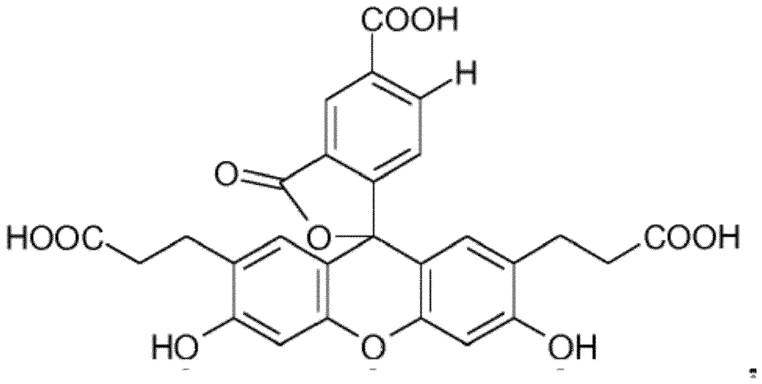
das 2', 7'-bis(2-Carboxyethyl)-6-Carboxyfluorescein der Formel: 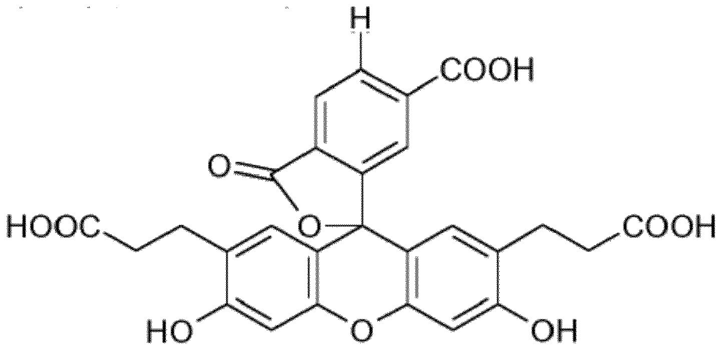
das 2', 7'-bis(2-Carboxy-1,2-Dehydroethyl)-5-Carboxy-Fluorescein der Formel: 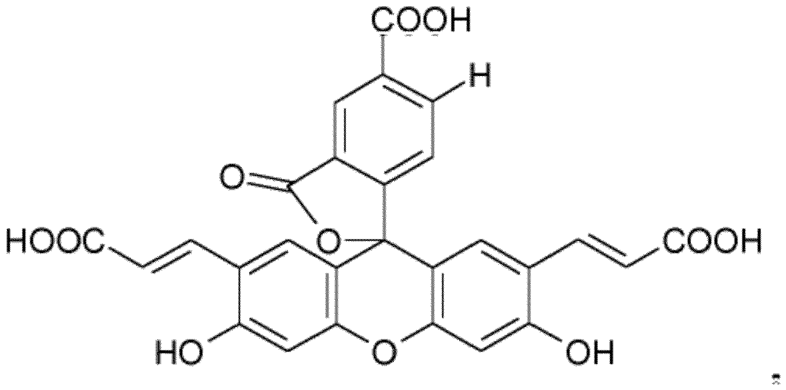
und das 2', 7'-bis(2-Carboxy-1,2-Dehydroethyl)-6-Carboxy-Fluorescein der Formel: 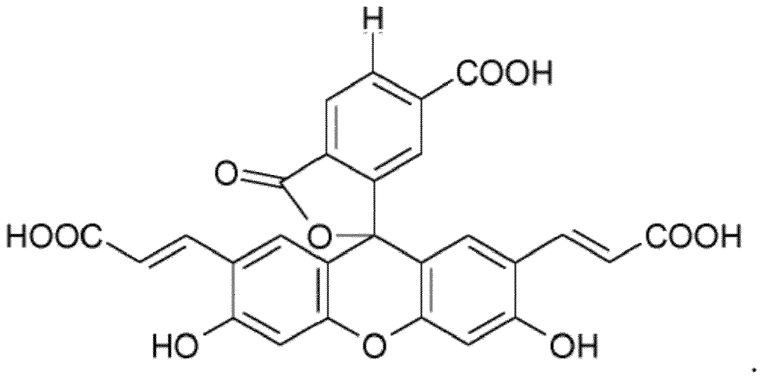
**dadurch gekennzeichnet, dass** das genannte Verfahren eine spezifische Esterifizierungsstufe der nicht aromatischen Carbonsäurefunktionen einer Verbindung umfasst, die der Formel (Ic) entspricht, welche den Erhalt von Esterderivaten der genannten Verbindung der Formel (Ic) zulässt, deren nicht aromatische Carbonsäurefunktionen mit einer isomerenreinheit Position von weniger als 95 % esterifiziert sind, gefolgt von einer Trennungsstufe des genannten Esterderivats, dann eventuell einer Verseifungsstufe des genannten Esterderivats.

2. Zubereitungsverfahren gemäß Anspruch 1, **dadurch gekennzeichnet, dass** es umfasst:
- eine Kondensierungsstufe der Verbindung der Formel (II) 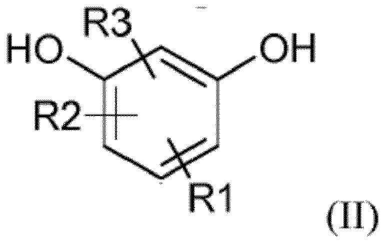
- in der R1 und R3 H darstellen,
und R2 eine Gruppe -A-COOH darstellt, wobei A eine Gruppierung (CH₂)₂- oder -CH=CH- darstellt,
mit einer Verbindung der Formel (V) 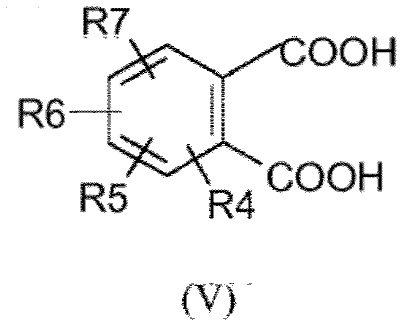
- in der R4, R5, R7 den Wasserstoff darstellt und R6 eine Gruppe -COOH darstellt,
um ein Zwischenprodukt der Formel (IX) zu erhalten: 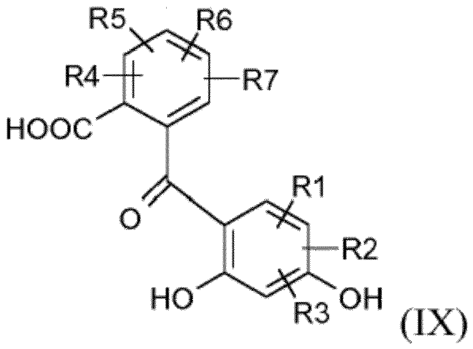 in der R1, R2, R3, R4, R5, R6, R7 der voranstehenden Definition entsprechen,
- eine Reinigungsstufe des Zwischenprodukts der Formel (IX) per Waschen und / oder Rekristallisation,
- eine Kondensierungsstufe der Zwischenverbindung der Formel (IX) mit einer Verbindung der Formel (II), um die Verbindung zu erhalten der Formel: 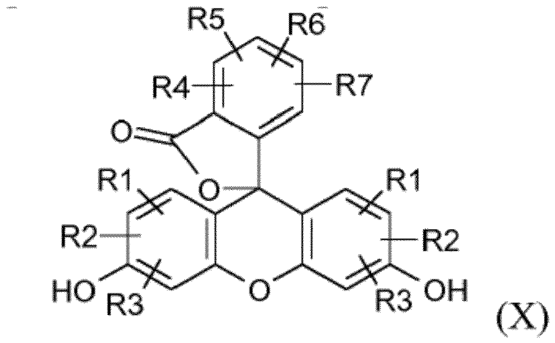 in der R1, R2, R3, R4, R5, R6 und R7 der voranstehenden Definition entsprechen;
- eine spezifische Esterifizierungsstufe der Verbindung der Formel (X) mit der Verbindung der Formel R8-OH (III), in der R8 eine Gruppe darstellt, die ausgewählt ist aus der Gruppe, umfassend:
∘ die linearen oder verzweigten Alkylgruppen in C₁ bis C₃₀ und / oder die eine oder mehrere doppelte oder dreifache Bindung(en) umfassen können, die eventuell durch einen oder mehrere Substituenten substituiert sind, die ausgewählt sind aus der Gruppe, umfassend die Halogen-, Hydroxy-, Nitro-, Amino-Gruppen und -COOR, in der R eine Alkylgruppe in C₁ bis C₃₀ ist,
∘ Cykloalkyle in C₃ bis C₁₂,
∘ lineare oder verzweigte Alkyloxyalkylene in C₁ bis C₃₀
∘ Aryle, die eventuell durch eine oder mehrere Gruppen substituiert sind, welche aus den Halogen-, Hyroxy- und Estergruppen ausgewählt sind,
∘ Aryloxyalkylene in C₁ bis C₃₀,
∘ Aryl-Alkyle in C₁ bis C₃₀,
um eine selektive Esterifizierung der nicht aromatischen Carbonsäurefunktionen zu realisieren und die Zwischenverbindung der Formel (VIII) zu erhalten: 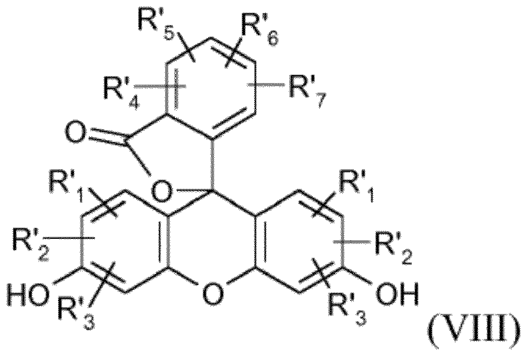
- in der R'1, R'3, R'4, R'5, R'6 und R'7, in der R'2 eine Gruppierung -A-COO-R8 ist, die A und R8 gemäß der voranstehenden Definition, ähnlich oder von ihr unterschiedlich sind, wobei wenigstens eine der Gruppierungen A von einer einfachen Bindung unterschiedlich ist und R'1 und R'3 einen Wasserstoff darstellen,
und in der R'6 eine Gruppierung -COOH ist und R'4, R'5 und R7 einen Wasserstoff darstellen,
- eine Trennstufe der Positionsisomere der Verbindung der Formel (VIII) durch Waschen und / oder Rekristallisation ist,
- eine Verseifungsstufe der Esterfunktionen, die die Regeneration der Verbindung der Formel (Ic) gemäß Definition in Anspruch 1 in Form eines einzigen Positionsisomers mit einer isomerenreinheit Position von mehr als oder gleich 95 % zulässt.

3. Verfahren gemäß Anspruch 1 bis 2, **dadurch gekennzeichnet, dass** die spezifische Esterifizierungsstufe der nicht aromatischen Carbonsäurefunktionen eine Reaktion mit BF₃ ist, das in einem primären oder sekundären Alkohol bei einer Temperatur von 10° C bis 40° C aufgelöst ist.

4. Verfahren gemäß Anspruch 3, **dadurch gekennzeichnet, dass** der primäre oder sekundäre Alkohol Methanol, Ethanol oder Isopropanol ist.

## Claims

1. Method for preparation of compounds with isomeric position purity of at least 95% according to formula (Ic) chosen from the group including 2',7'-bis(2-carboxyethyl)-5-carboxyfluoresceine of formula: 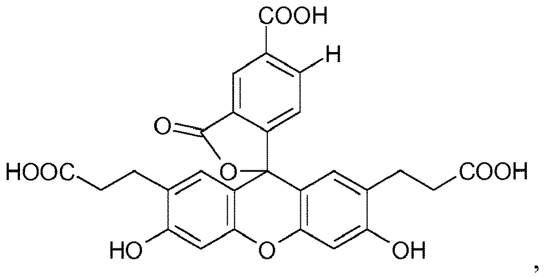 2',7'-bis(2-carboxyethyl)-6-carboxyfluoresceine of formula: 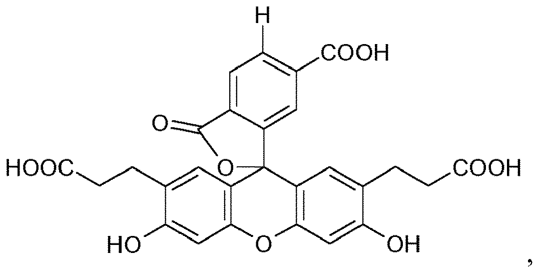 2',7'-bis(2-carboxy-1,2-dihydroethyl)-5-carboxy-fluoresceine of formula: 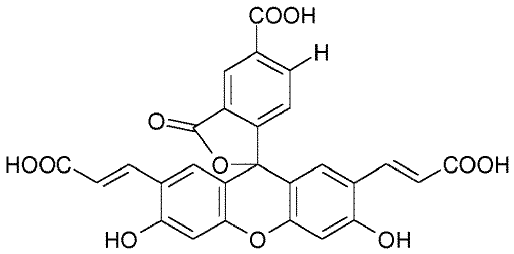 and, and 2',7'-bis(2-carboxy-1,2-dihydroethyl)-6-carboxy-fluoresceine of formula: 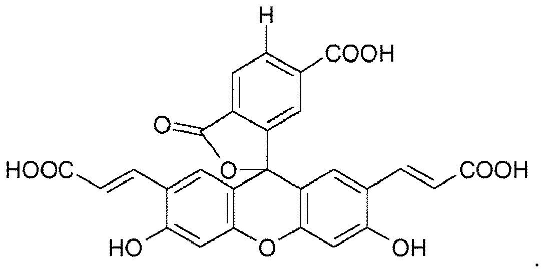 **characterised in that** said method comprises a specific step for esterification of non-aromatic carboxylic acid functions of a compound according to formula (Ic) by which an ester derivative of said compound of formula (Ic) can be obtained in which the non-aromatic carboxylic acid functions are esterified with an isomeric position purity of less than 95%, followed by a separation step of said ester derivative, and then possibly a saponification step of said ester derivative.

2. Preparation method according to claim 1, **characterised in that** it comprises:
- a condensation step of the compound of formula (II) 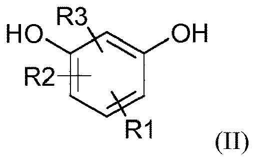
- in which R1 and R3 represent H, and R2 represents an -A-COOH group where A represents a -(CH₂)₂- or -CH=CH- group,
with a compound of formula (V) 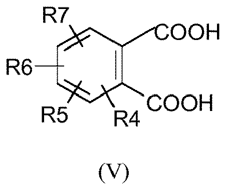
- in which R4, R5, R7 represent hydrogen, and R6 represents a -COOH group,
to obtain an intermediary of formula (IX); 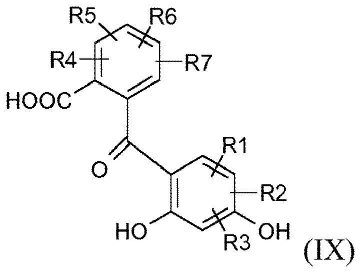 in which R1, R2, R3, R4, R5, R6 and R7 are as defined above,
- a step to purify the intermediary of formula (IX) by washing and/or recrystallisation
- a step to condense the intermediate compound of formula (IX) with a compound of formula (II) to obtain the compound of formula: 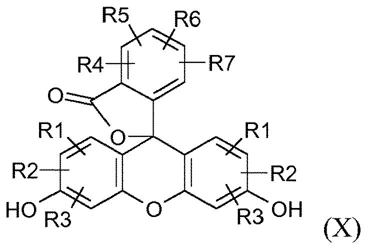 in which R1, R2, R3, R4, R5, R6 and R7 are as defined above,
- a step for specific esterification of the compound of formula (X) with the compound of formula R8-OH (III) where R8 represents a group chosen from the group comprising:
∘ linear or ramified alkyl groups in C₁ to C₃₀ and/or possibly comprising one or several double or triple bonds, possibly substituted by one or several substituents chosen from the group comprising halogeno, hydroxyl, nitro, amino and -COOR groups where R is an alkyl group in C₁ to C₃₀,
∘ cycloalkyls in C₃ to C₁₂,
∘ linear or ramified alkyloxyalkylenes in C₁ to C₃₀,
∘ aryls, possibly substituted by one or several groups chosen from among the halogeno, hydroxyl and ester groups,
∘ aryloxyalkylene in C₁ to C₃₀,
∘ aryl-alkyl in C₁ to C₃₀,
to make selective esterification of non-aromatic carboxylic acid functions and to obtain the intermediate compound of formula (VIII): 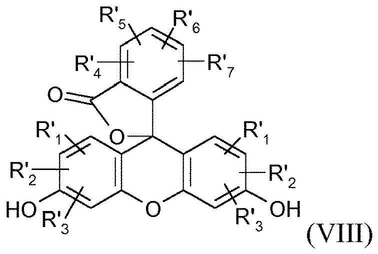
- in which R'1, R'3, R'4, R'5, R'6 in which:
- R'2 is a similar or different -A-COO-R8 group, where A and R8 are as defined above, at least one of the A groups being different from a single bond, and R'1 and R'3 represent a hydrogen,
and in which R'6 is a -COOH group and R'4, R'5 and R'7 represent a hydrogen,
- a step to separate position isomers from the compound of formula (VIII) by washing and/or recrystallisation,
- a saponification step of the ester functions to regenerate the compound of formula (Ic) as defined in claim 1 in the form of a single position isomer with isomeric position purity equal to 95% or more.

3. Method according to either claim 1 or 2, **characterised in that** the specific esterification step of non-aromatic carboxylic acid functions is a reaction with BF₃ dissolved in a primary or secondary alcohol, at a temperature of between 10°C and 40°C.

4. Method according to claim 3, **characterised in that** the primary or secondary alcohol is methanol, ethanol or isopropanol.
